# EUROPEAN PATENT APPLICATION

(11) **EP 2 799 086 A1**
(43) Date of publication of application: **05.11.2014**
(21) Application number: 13162989.1
(22) Date of filing: 09.04.2013
(51) Int. Cl.: A61K 39/104, C07K 16/12

(54) **Neuraminidase-based vaccine compositions and methods for diagnosing and preventing pneumococcal diseases**

(71) Applicant: Chang Gung Medical Foundation, Linkou Branch, Taoyuan County 333 (TW)
(72) Inventor: Chiu, Cheng-Hsun, 333 Taoyuan County (TW); Janapatla, Rajendra Prasad, 333 Taoyuan County (TW); Chen, Chyi-Liang, 333 Taoyuan County (TW); Hsu, Mei-Hua, 333 Taoyuan County (TW); Chen, Hsiu-Ling, 333 Taoyuan County (TW); Huang, Chung-Tsui, 333 Taoyuan County (TW); Chang, Hsin-Ju, 333 Taoyuan County (TW); Liao, Wan-Ting, 333 Taoyuan County (TW)
(74) Representative: Lang, Christian

(57) **Abstract**

A protein vaccine composition of protection against pneumococcal infection in a subject is disclosed. The vaccine composition comprises three recombinant pneumococcal neuraminidases: NanA, NanB, and NanC of *S. pneumoniae* strains CGSP14, wherein administration of the recombinant full-length- and partial-pneumococcal neuraminidases elicits an immune response to all serotypes of *S. pneumoniae.* In one embodiment, the method further includes a step of adding adjuvants to enhance the immune response. The method also comprises a step of using passive antibodies, wherein said passive antibodies are anti-neuraminidase antibodies generated from neuraminidases-immunized animals against NanA, NanB, and NanC. Meanwhile, this invention also provides a method for the molecular diagnosis of pneumococcal infection, and a method of detecting neuraminidase activity.

## Description

### FIELD OF THE INVENTION

The present invention relates to a neuraminidase-based vaccine composition for the development of vaccine to prevent pneumococcal diseases and a method for the diagnosis of the pneumococci-infected samples not just from urine but also from blood and pleural effusion in pyothorax. More particularly, this invention relates to a universal protein vaccine against a pneumococcal infection.

### BACKGROUND OF THE INVENTION

*Streptococcus pneumoniae* is one of Gram-positive encapsulated diplococci. Pneumococcal infection is a leading infectious cause of high mortality and morbidity worldwide, especially among young children below two years of age and the elderly over sixty years of age. Globally, pneumococcal infections are estimated to cause about 1.6 million deaths annually, including 1 million children less than five years old. Even though certain vaccines have been applied to prevent the *S*. *pneumoniae* infection, the mortality rate caused by this organism still ranked the highest. The spectrum of the *S. pneumoniae-*related diseases includes invasive pneumococcal disease (IPD), such as sepsis and meningitis; lower respiratory infections, such as bacterial pneumonia; and upper respiratory infections, such as acute otitis media (AOM) (Tuomanen *et al.,* 1995).

According to the reports of World Health Organization (WHO) in 2005, acute respiratory tract infections were the major cause of death globally, in which the deaths were chiefly attributable to the *S. pneumoniae*-associated community-acquired pneumonia (CAP). This threatening issue strongly raises the urgency for both diagnosis and prevention. Although the diagnoses of pneumococci have been developed for decades, conventional culture methods that are tedious and time-consuming, to proliferate enough bacteria for specific and sensitive detection are still heavily relied on. Therefore, based on specific DNA amplification and antigen detection, the tests of non-culture samples from sputum, urine, and blood have been continuously developed over time in order to identify pneumococci as the etiological agent of diseases. However, the consequences of those tests were always unsatisfactory in certain applications. For instance, the application of PCR testing for the diagnosis of IPD has ever shown to be sensitive enough when using blood or urinary samples, and has poorly specific when using respiratory samples. To overcome the problem of poor specificity when using sputum samples, recently a dual-PCR testing protocol using pneumococcal *IytA* and *ply* as targets has been successfully developed and evaluated.

Another disappointing aspect for diagnosis revealed that only one third of pathogens could be recovered from patient's sputum when using conventional culture methods. In addition, the controversial results lacked specificity correlated to CAP because nasopharyngeal carriage of pneumococci could be found both in healthy individuals and inadequate sputum samples. Though the etiological pathogens of CAP tested from blood culture and pleural fluid were specific, the positive rates were lower (<30%) compared to those of from sputum samples. For this reason, the development of antigen detection was applied to compensate the drawback of low specificity. Higher sensitivity of the pleural test, compared to pleural cultures, indicated that antigen detection for pleural samples could be a better application for the CAP study of pneumococcal etiology. Also, the detection of BinaxNOW pneumococcal C-polysaccharide in a urine sample with CAP showed unsatisfied result due to its high rate of false positive.

Currently, there are two kinds of vaccines, 23-valent pneumococcal polysaccharide vaccine (PPV23) and 7-valent pneumococcal conjugate vaccine (PCV7), available for general protection against potential IPD-causative pathogen strains. Vaccine PCV7 can target seven serotypes, including 4, 6B, 9V, 14, 18C, 19F, and 23F. Before the introduction of PCV7, the PCV7-targeted 7 serotypes (4, 6B, 9V, 14, 18C, 19F, and 23F) were responsible for about 90% of incidence of IPD in young children in the United States and for more than 60% of those in Europe. After the introduction of PCV7 vaccination, the cases of IPD in children less than 5 years old declined by 56% in 2001 and by 76% in 2004. In contrast, PPV23 vaccination seems to difficultly reach firm conclusions in clinical effectiveness (around 50-70% effective). Although two doses of PCV7 and following one dose of PPV23 were recommended to broaden protection, the effectiveness of vaccines was significant on the protection of those seven PCV7-covered serotypes rather than others. The results suggested that PPV23 seems not necessary as a boost dose for broadening protection.

At least 93 different polysaccharide (PS) capsules of *S. pneumoniae* have been verified to be specific serotypes, and further classified to be 46 serogroups. Among all pathogenic pneumoncocci worldwide, serotype 14 and serogroup 6 are predominant. In addition, the majority of IPD is generally caused by about 15 serotypes. However, only a few antimicrobial resistant pneumococcal clones could spread fast. The incidence of antimicrobial resistance of pneumococci varies regionally, and is associated with the spectrum of antibiotic use, population density, the indigenous prevalence of resistant strains, ages and time. Although the resistance patterns have been shown to be different around the world, the predominant serotypes commonly identified are 6A/B, 9V, 14, 19A/F, and 23F. Based on epidemiological study, the nasopharyngeal (NP) carriage of predominant pneumococci has been observed in many young children, indicating that NP carriage may play an important role in pneumococcal transmission, especially for antibioticresistant strains.

Despite effective reduction of the incidence of IPD caused by vaccine serotypes in both children and adults due to the usage of the current pneumococcal vaccine PCV7, the mortality rate of pneumococcal disease remains high. After the introduction of PCV7 in 2000, nonvaccine serotype 3 was found to be a significant cause for necrotizing pneumonia in children in Utah, whereas a mucoid serotype 3 was usually reported to cause lung abscess in adults. Serotype 19A has been reported the predominant serotype causing IPD all over the world. In Taiwan, complicated pneumococcal pneumonia still remains a clinically intricate problem, and its significant association with the clonal spread of CC320 within serotype 19A was noteworthy recently in Taiwan. Besides pneumococcal serotypes 3 and 19A, other nonvaccine serotypes, including 1, 5, 6A, and 7F, were also common causes for IPD around the world (Grijalva and Pelton, 2011). A second-generation 13-valent pneumococcal conjugate vaccine (PCV13) was therefore developed to address this new global issue of pneumococcal infection in 2010 (Grijalva and Pelton, 2011).

Hemolytic uremic syndrome (HUS), one of the most severe complications of IPD, mainly occurs in children, and it is also associated with hemolytic anemia, thrombocytopenia, and acute renal failure. This disorder, usually occurring in healthy young children, is one of the most common causes of acute renal failure in pediatric patients. Management of the pneumococcal HUS primarily includes an intensive antimicrobial therapy and the dialysis and transfusion of washed RBC, platelets and plasma. Most cases of HUS are reported by an acute gastroenteritis related to *Escherichia coli* (O157:H7) and often show good prognosis with recovery of renal function. However, the mortality rate of patients with pneumococcal HUS was high in early reports. Of the 14 cases recently reported from USA, 1 (7%) died and 4 (29%) developed chronic kidney disease.

*S. pneumoniae* encodes many virulence factors, but only the secreted neuraminidase A (NanA) was reported to be attributed to HUS. Neuraminidase cleaves *N-*acetylneuraminic acid (sialic acid) residues on red blood cells (RBC), platelets and endothelial cells, and the results may lead to the exposure of the Thomsen-Friedenrich antigen (T antigen), and allow the circulating anti-T antigen antibodies to react with the exposed T antigen on cells. The role of neuraminidase(s) in pneumococcal diseases is illustrated based on the fact that pneumococci produce two or three distinct neuraminidases, which are NanA, NanB, and NanC. All three neuraminidases have typically signal peptides for secretion, wherein NanA, unlike NanB and NanC, contains a C-terminal cell surface anchorage domain. NanA and NanB expose host cell surface receptors for pneumococcal adherence by cleaving sialic acid from the glycans and mucin of cell surface, and thereby it promotes the pneumococcal colonization on the upper respiratory tract. In *in vivo* study, a NanA mutant was cleared from the nasopharynx, trachea, and lungs within 12 hours postinfection, while a NanB mutant persisted but did not increase in either the nasopharynx, trachea, or lungs. However, the role of NanC remains unknown.

The nonvaccine serotypes have been emerging after the use of vaccines. Moreover, nothing worse than the fact that nonvaccine strains usually displayed increase antimicrobial resistance and virulence. This is the reason why the issue of pneumococcal infection remains to be a global public health challenge. Thus, continued efforts to develop new diagnostic methods and to develop vaccines with expanded or universal coverage, such as a universal protein vaccine, are critically required for the better control of the pneumococcal infections.

### SUMMARY OF THE INVENTION

Based on the finding that *S. pneumoniae* isolates causing HUS were mostly found to produce all of the three neuraminidases, including NanA, NanB, and NanC, three primer sets were designed to detect and clone these pneumococcal neuraminidase genes in this invention. The three recombinant neuraminidases combined together serve as an ideal vaccine candidate because it presents the best protective efficacy against pneumococcal infection in mice, compared to the others. In one embodiment, the present invention provides a method of molecular detection of pneumococcal diseases by PCR in a *S. pneumoniae*-infected sample to amplify three neuraminidase genes based on the sequences of the three neuraminidase genes of *S. pneumoniae* strain CGSP14. In another embodiment, the present invention provides a vaccine composition generating immunization in humans and animals against *S. pneumoniae* infection, and the composition comprises the three recombinant neuraminidases, including NanA, NanB, and NanC.

In still another embodiment, the present invention provides protection against *S. pneumoniae* infection using three pneumococcal neuraminidases as antigens in active immunization, and/or using anti-neuraminidase antibodies for passive immunization. In a further embodiment, the present invention is able to detect the presence of any of the three neuraminidases in the *S. pneumoniae*-infected samples using the anti-neuraminidase antibodies generated from neuraminidases-immunized animals. In still a further embodiment, the present invention provides a method of detecting inhibition of a neuraminidase activity by antibody or antiserum using flow cytometry.

### BRIEF DESCRIPTION OF THE DRAWINGS

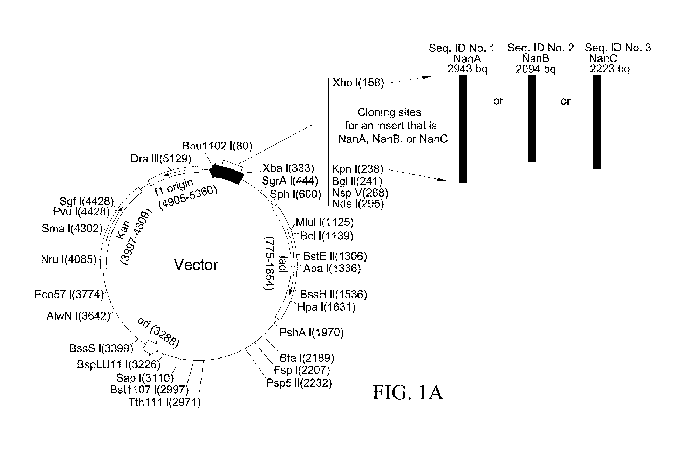

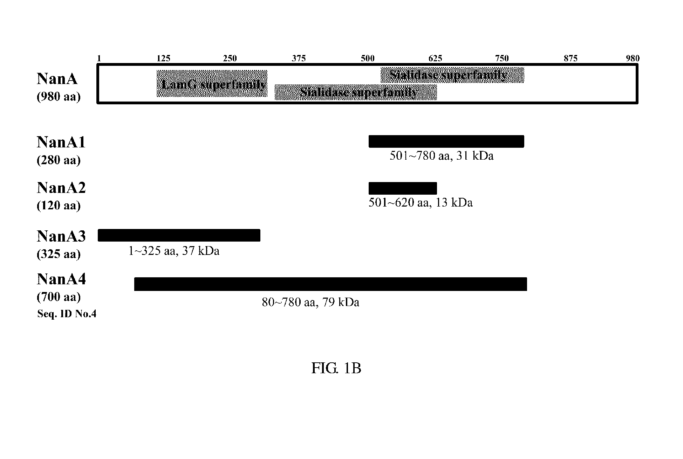

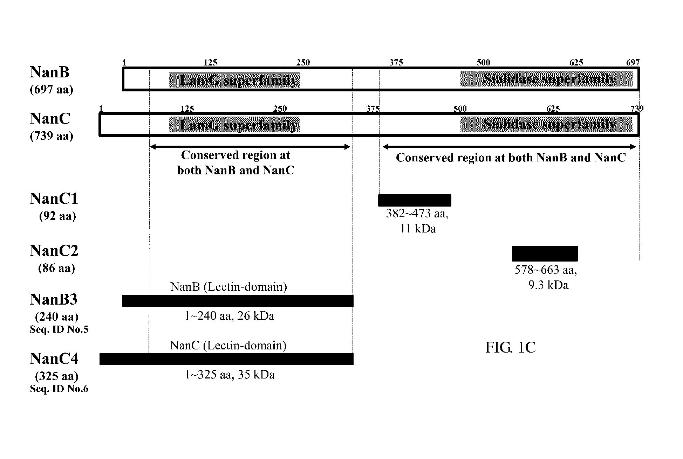

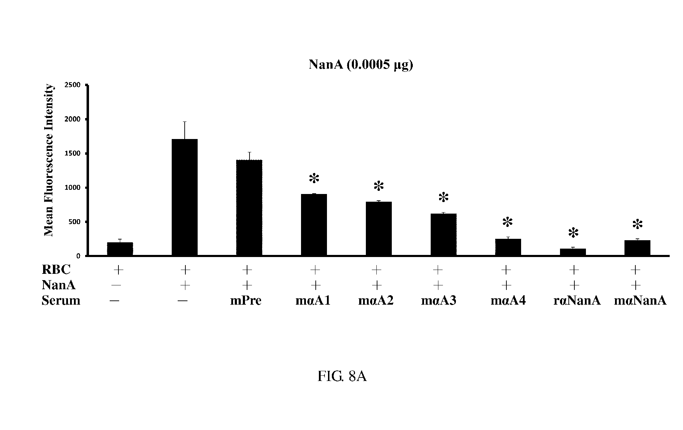

Figures 1A-1C: Schematic diagram of the recombinant neuraminidase clones and sub-clones. Each PCR-amplified amplicon of full-length-neuraminidase genes (including *nanA, nanB,* and *nanC* (Seq. ID Nos. 1 to 3)) (Figure 1A) and those partial neuraminidase genes (Figures 1B and 1C), which sequences were based on the genomic sequence of *S. pneumoniae* strain CGSP14, was cloned into an expression vector, such as pET29b, through restriction enzyme digest by *Kpn*I and *Xho*I*.* Seq. IDs (No. 1, No. 2, and No. 3) indicate individual sequences for those full-length-genes (*nanA, nanB*, and *nanC*, respectively). Eight sub-clones containing the conserved regions of Laminin G (LamG, a lectin domain) superfamily or sialidase superfamily or both, including NanA1, NanA2, NanA3, and NanA4 belonging to a series of NanA sub-clones (Figure 1B), NanB3 belonging to a NanB sub-clone, and NanC1, NanC2, and NanC4 belonging to a series of NanC sub-clones are illustrated (Figure 1C). Seq. IDs (No. 4, No. 5, and No. 6) indicate individual sequences for those sub-clones (*nanA4, nanB3*, and *nanC4,* respectively) with sufficient inhibition efficacy equivalent to full-length-neuraminidase genes as shown in Figures 8A-8C.

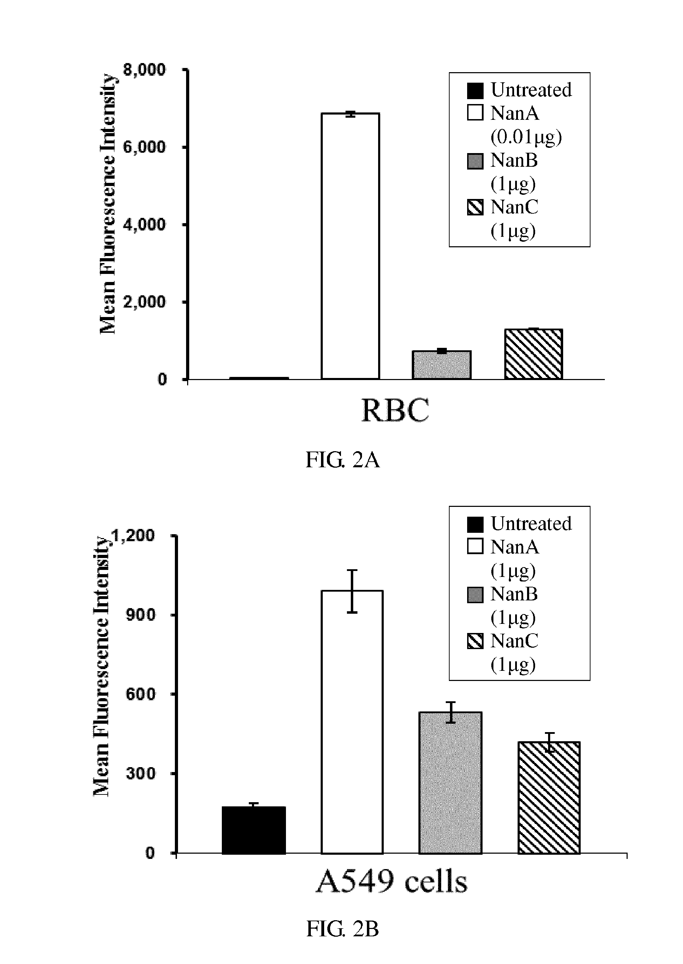

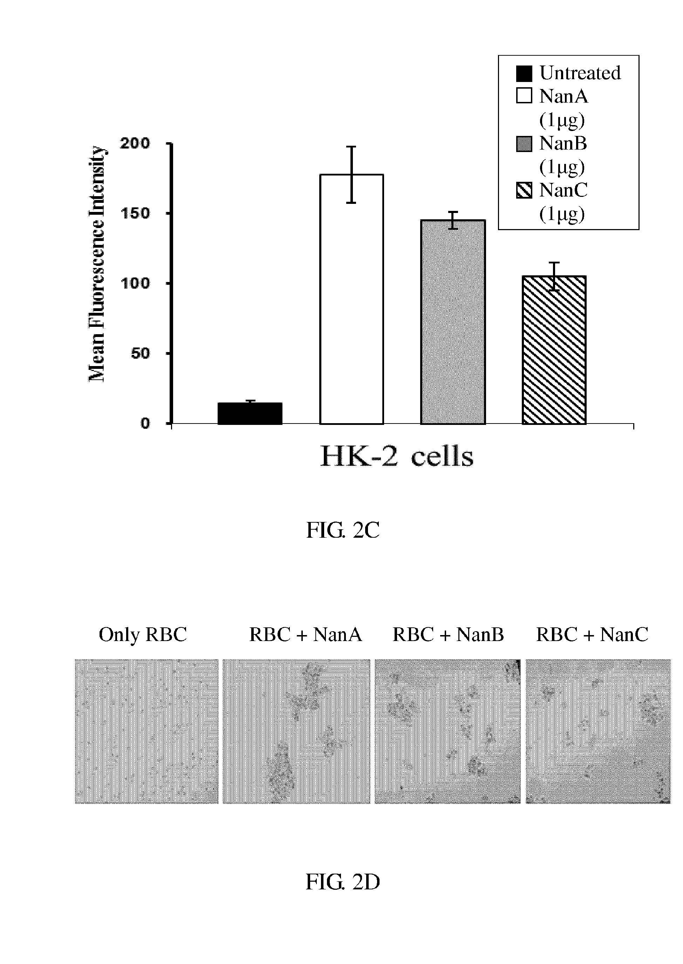

Figures 2A-2D: Thomsen-Friedenrich antigen (TA) exposure on cells. PNA lectin binding was used to detect the TA by flow cytometry. Numbers indicate fluorescence counts of samples, which are untreated cells (black), NanA-treated (white), NanB-treated (grey), and NanC-treated (hatched). NanA (0.01 µg), NanB (1 µg) and NanC (1 µg) can expose TA on RBC (Figure 2A). NanA, NanB and NanC (all were 1 µg) can expose TA on A549 (Figure 2B) and HK-2 cells (Figure 2C). Twenty µL aliquots of fluorescein isothiocyanate(FITC)-conjugated PNA lectin labeled RBC used for flow cytometric analysis were incubated at 37°C and observed under microscope to verify agglutination. Agglutination of RBC was observed when treated with NanA, NanB and NanC (0.1 µg) (Figure 2D).

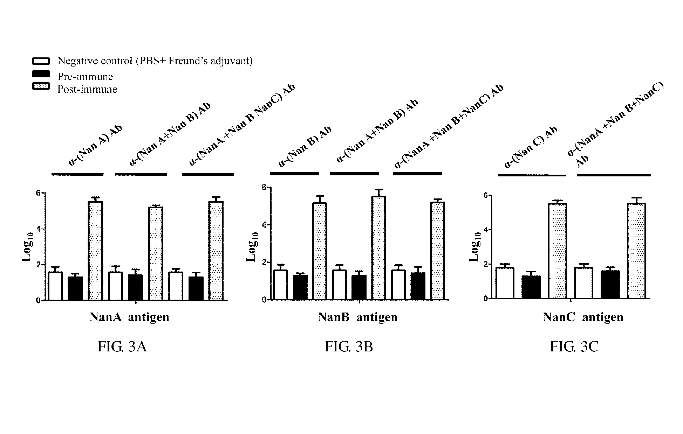

Figures 3A-3C: Confirmation of mouse polyclonal antisera against neuraminidase(s) by enzyme-linked immunosorbent assay (ELISA). Mouse post-immune (grey box) antisera against neuraminidase antigens NanA (Figure 3A), NanB (Figure 3B) and NanC (Figure 3C) from different combinations, including individual neuraminidase (NanA, NanB, or NanC), neuraminidase A+B (NanA + NanB), and neuraminidase A+B+C (NanA + NanB + NanC), were tested by ELISA, while the sera from pre-immune (black box) and negative control (only PBS plus Freund's adjuvant without antigen; white box) were also examined. The antigen-antibody interactions were quantified by using the peroxidase-conjugated goat anti-mouse IgG (Sigma) as a secondary antibody and tetramethylbenzidine/peroxide (R&D Systems, Minneapolis, MN, USA) as a color-developing substrate under the analysis of ELISA reader with the maximum absorbance band at a wavelenghth of 405 nanometer (EMax, Molecular Devices, Sunnyvale, CA 94089 USA). The value was presented by the logarithm of the value on y-axis.

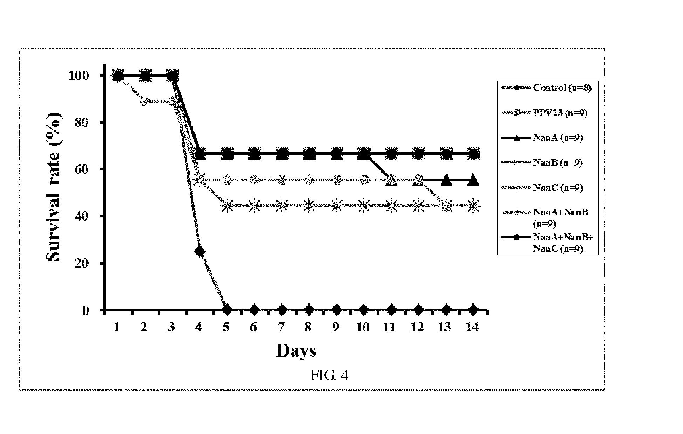

Figure 4: Vaccination tests. The individual or combination of three neuraminidases, NanA, NanB and NanC with 10 µg of each enzyme and about 0.5-mg neuraminidase/kg body weight, were applied as antigens to immunize mouse (BALB/C, one month old) four times at 2-week interval, while PPV23 vaccine and a negative control (PBS + Freund's adjuvant) were taken for comparison. Thereafter, *S. pneumoniae* serotype 3 (3x10³ cfu) used to challenge those neuraminidase-immunized mice was injected in mice tails intravenously. Freund's complete adjuvant for the first time immunization and Freund's incomplete adjuvant for the last three immunizations were used with the ratio of 1:1 to the antigen(s). Mice survival rate (%) was determined during 14 days feeding after four times neuraminidase immunization and a subsequent *S. pneumoniae* challenge. N is the number of mice for test.

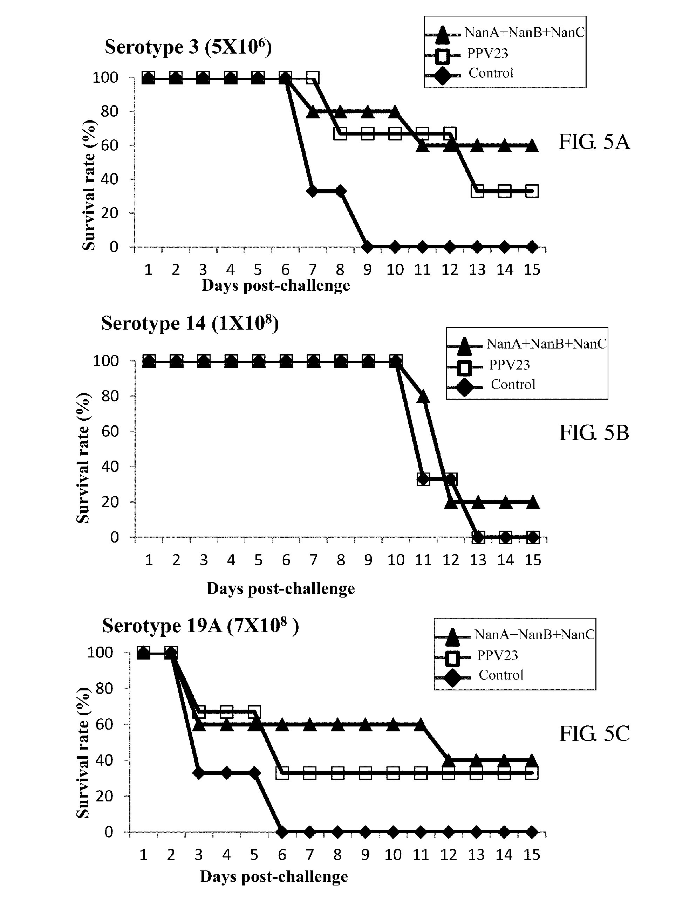

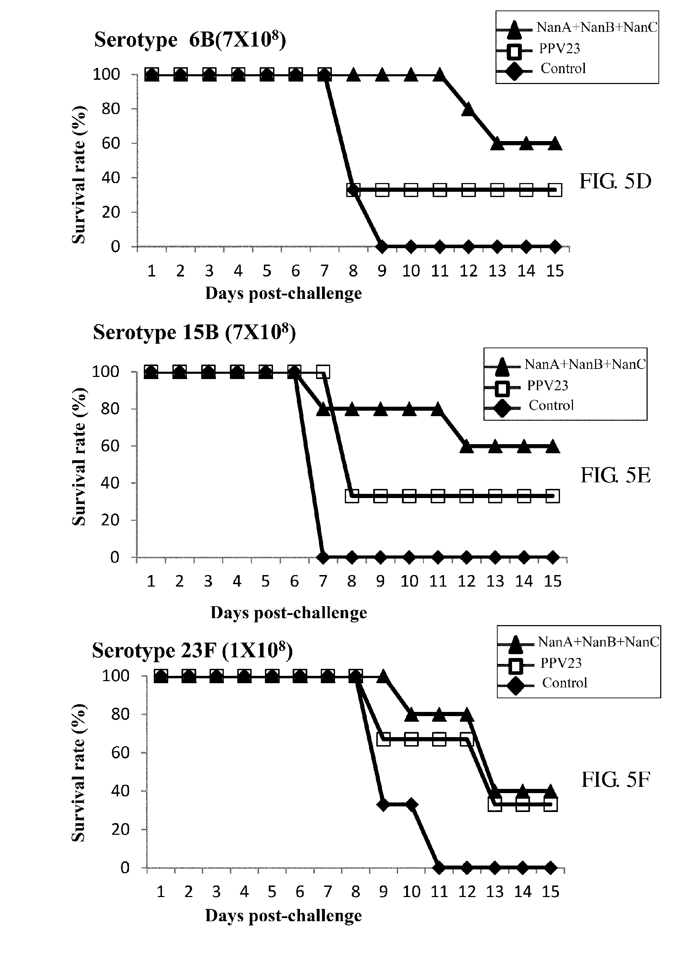

Figures 5A-5F: Protective effect of vaccination on different serotypes. The individual or combination of three neuraminidases, NanA, NanB and NanC with 10 µg of each enzyme, were applied as antigens to immunize mice (BALB/C, one month old, n = 5) four times at 2-week interval, while PPV23 vaccine (n = 3) and a negative control (PBS + Freund's adjuvant, n = 3) were taken for comparison. Thereafter, *S. pneumoniae* serotype 3 (5x10⁶ cfu) (Figure 5A), serotype 14 (1x10⁸ cfu) (Figure 5B), serotype 19A (7x10⁸ cfu) (Figure 5C), serotype 6B (7x10⁸ cfu) (Figure 5D), serotype 15B (7x10⁸ cfu) (Figure 5E), or serotype 23F (1x10⁸ cfu) (Figure 5F) was used to challenge those neuraminidase-immunized mice. Freund's complete adjuvant for the first time immunization and Freund's incomplete adjuvant for the last three immunizations were used with the ratio of 1:1 to the antigen(s). Mice survival rate (%) was determined during 14 days after four times neuraminidase immunization and a subsequent *S. pneumoniae* challenge. N is the number of mice for test.

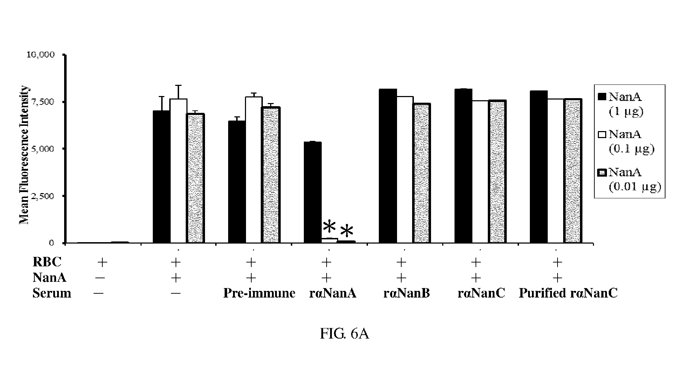

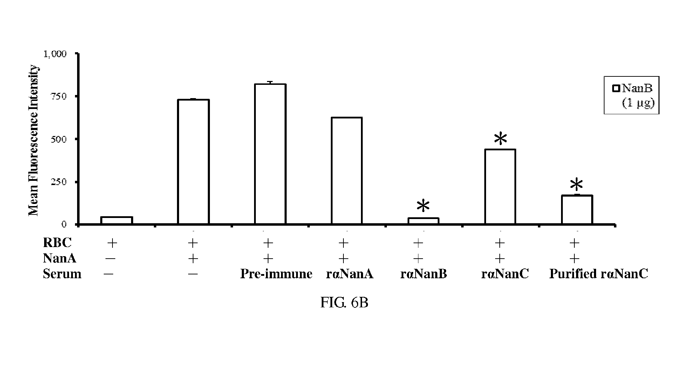

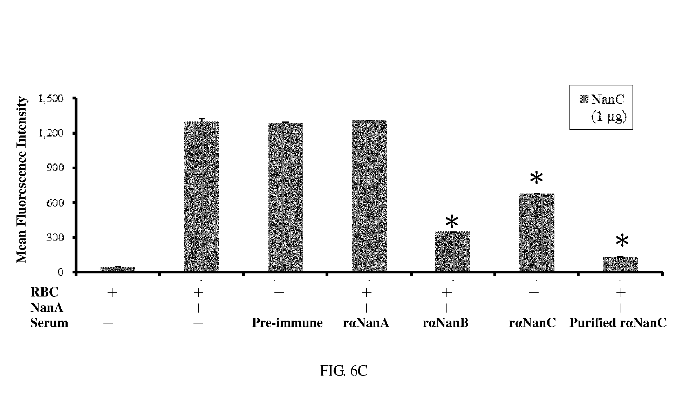

Figures 6A-6C: Inhibition of neuraminidase activity by the anti-serum raised from neuraminidase-immunized rabbit. Neuraminidase-mediated TA antigen exposure presented on RBC cells was quantified by flow cytometry analysis, where FITC-labeled PNA Iactin was used for the recognition of TA antigen. Prior to the quantification of TA exposure, an individual neuraminidase (including NanA, NanB, and NanC) was individually added for the treatment with different anti-neuraminidase anti-sera (30 µg/mL), including purified anti-NanC antiserum. (Figure 6A) NanA added for the treatment of rabbit anti-neuraminidase anti-serum (raNanA) were 1 µg (black bars), 0.1µg (white bars), and 0.01µg (grey bars). (Figure 6B) NanB added for the treatment of rabbit anti-NanB anti-serum (rα NanB) was 1 µg (white bars). (Figure 6C) NanC added for the treatment of rabbit anti-NanC anti-serum (raNanC) was 1 µg (grey bars). * indicates p<0.05, when compared to the controls (only neuraminidase addition without serum treatment; neuraminidase addition with pre-immune serum treatment).

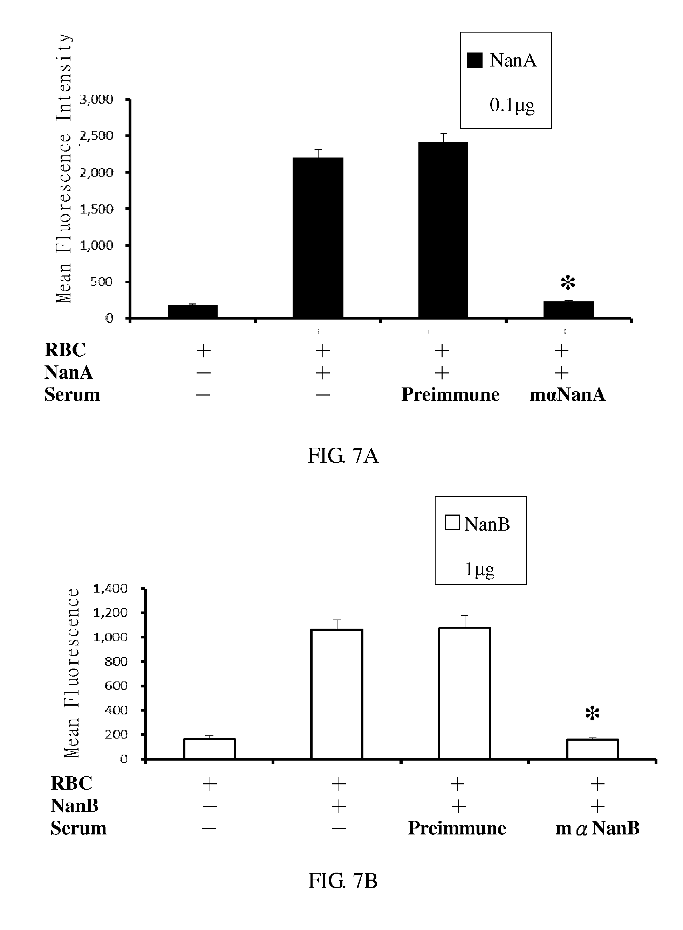

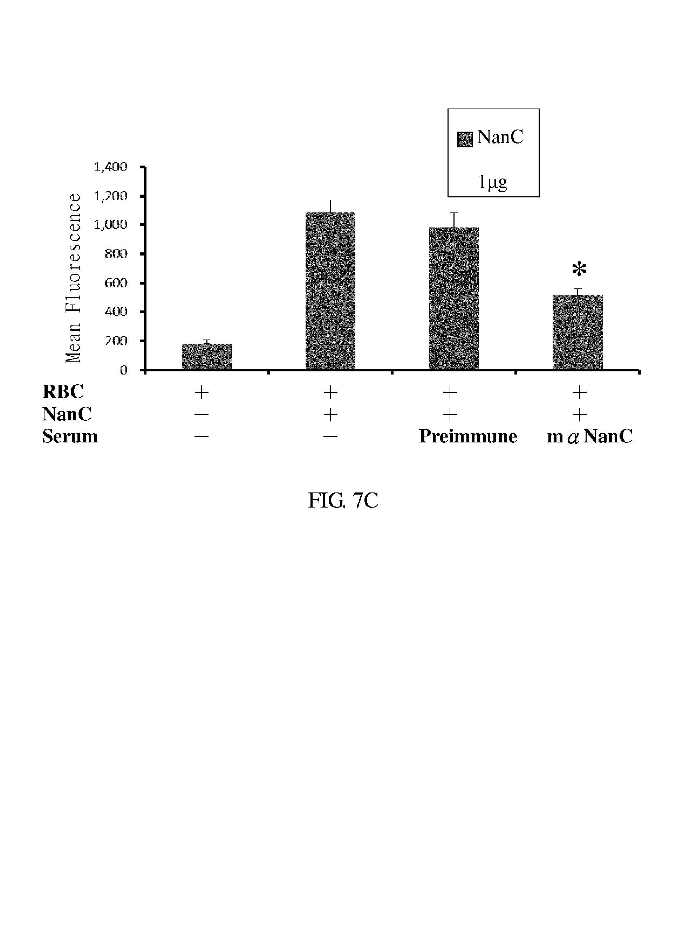

Figures 7A-7C: Inhibition of neuraminidase activity by the anti-serum raised from neuraminidase-immunized mouse. Neuraminidase-mediated TA antigen exposure presented on RBC cells was quantified by flow cytometry analysis, where FITC-labeled PNA Iactin was used for the recognition of TA antigen. Prior to the quantification of TA exposure, an individual neuraminidase (including NanA, NanB, and NanC) was added for the treatment with specific anti-neuraminidase anti-sera (30 µg/mL). (Figure 7A) NanA added for the treatment of mouse anti-NanA anti-serum (mαNanA) was 0.1 µg. (Figure 7B) NanB added for the treatment of mouse anti-NanB anti-serum (mαNanB) was 1 µg. (Figure 7C) NanC used for the treatment of mouse anti-NanC anti-serum (mαNanC) was 1 µg. * indicates p<0.05, when compared to the controls (only neuraminidase addition without serum treatment; neuraminidase addition with pre-immune serum treatment).

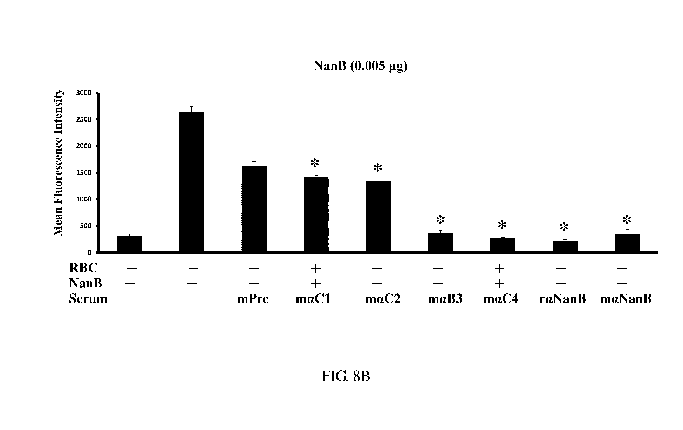

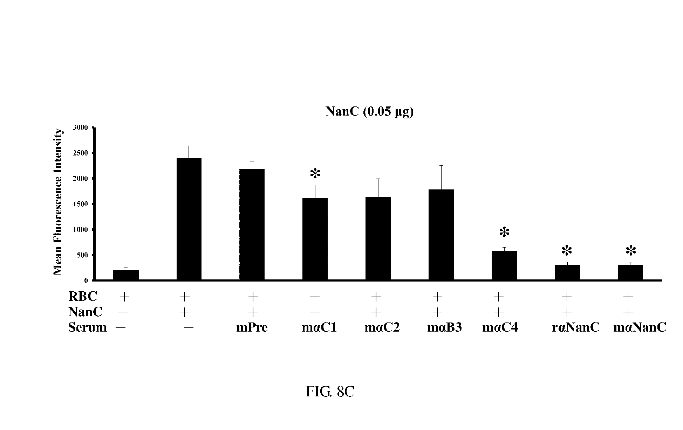

Figures 8A-8C: Comparison of the inhibition of neuraminidase activity using various anti-sera. This detection protocol was followed as described in Figures 6 and 7. Neuraminidase-mediated TA antigen exposure presented on RBC cells was quantified by flow cytometry analysis. Prior to the quantification of TA exposure, an individual neuraminidase, including NanA (Figure 8A), NanB (Figure 8B), and NanC (Figure 8C), was added for the treatment using different anti-neuraminidase anti-sera. The anti-sera obtained from full length of neuraminidases-immunized rabbits and mice (as described in Figures 6 and 7) are denoted as raNan(s) and mαNan(s), respectively. In addition, the anti-sera raised in various antigens-immunized mice, including NanA1, NanA2, NanA3, NanA4, NanB/C1, NanB/C2, NanB3, and NanC4 (as described in Figure 1B and 1C) are denoted as mαA1, mαA2, mαA3, mαA4, mαB/C1, mαB/C2, mαB3, and mαC4, respectively. * indicates *p* < 0.05, when compared to the control that is only neuraminidase without serum treatment. mPre: mouse pre-immune serum.

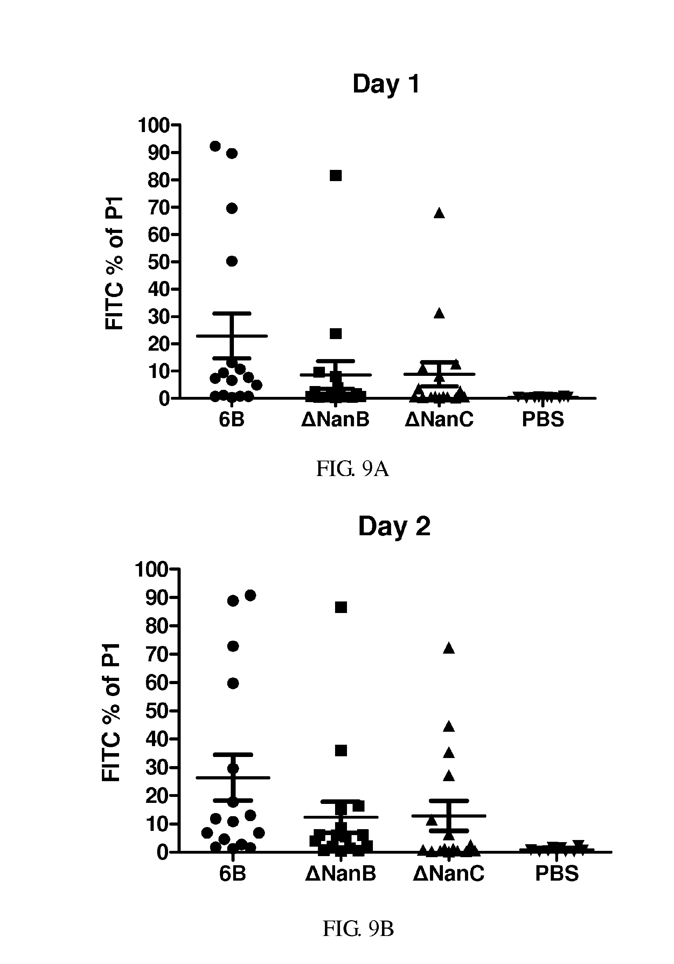

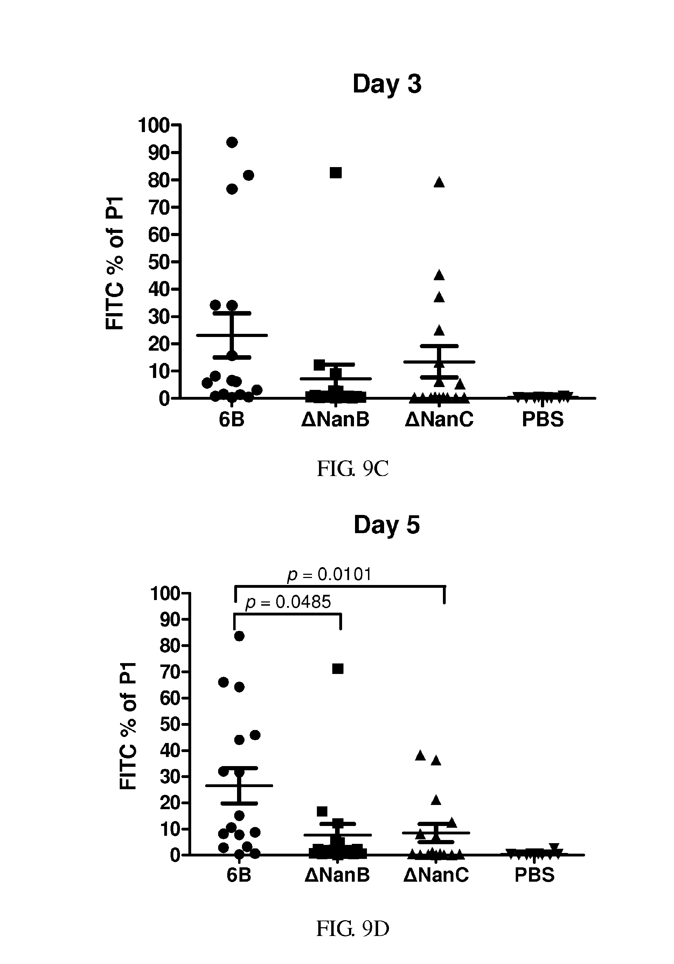

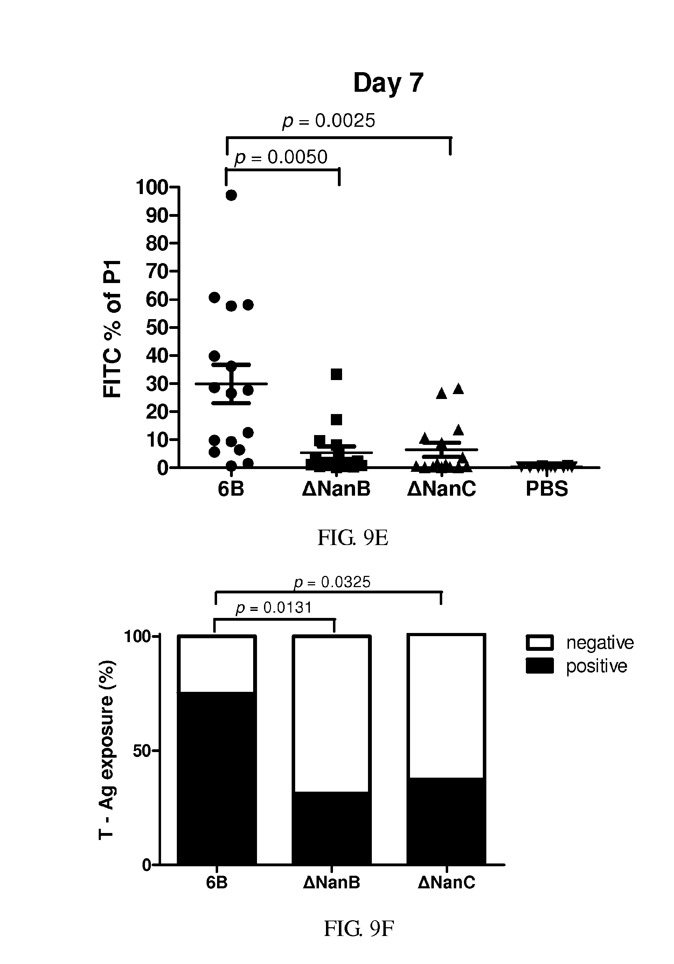

Figures 9A-9F: The influence of NanB and NanC on the exposure of T-antigen on the RBC during the infection of *S. pneumoniae.* BALB/c mice were intravenously challenged with each (10⁸ cfu) of wild type *S. pneumoniae, nanB* and *nanC* mutants. At specific time points, 1^{st} day (Figure 9A), 2^{nd} day (Figure 9B), 3^{rd} day (Figure 9C), 5^{th} day (Figure 9D) and 7^{th} day (Figure 9E), the blood samples were collected by retro-orbital puncture and examined by flow cytometer. Statistics by Paired *t* test; solid circle (•) shows wild type *S. pneumoniae* (6B); P1 means the gated region in the analysis of flow cytometry; solid squares (■) shows *nanB* mutant; solid triangles (A) shows *nanC* mutant; reverse solid triangles (▼) shows PBS; black line shows geometric mean; error bars represent standard deviation; n = 16 mice per each time point per each strain used. (Figure 9F) Based on the results at the 7^{th} day after challenge, the ratio of the mice with positive T-antigen exposure on RBC to the total tested mice was measured, and also, the ratio of mice with negative T-antigen exposure was presented. Statistics were measured by Chi-square test; black bar shows positive T-antigen exposure; white bar represents negative T-antigen exposure.

### DETAILED DESCRIPTION OF THE INVENTION

The detailed description set forth below is intended as a description of the presently exemplary device provided in accordance with aspects of the present invention and is not intended to represent the only forms in which the present invention may be prepared or utilized. It is to be understood, rather, that the same or equivalent functions and components may be accomplished by different embodiments that are also intended to be encompassed within the spirit and scope of the invention.

Unless defined otherwise, all technical and scientific terms used herein have the same meaning as commonly understood to one of ordinary skill in the art to which this invention belongs. Although any methods, devices and materials similar or equivalent to those described can be used in the practice or testing of the invention, the exemplary methods, devices and materials are now described.

All publications mentioned are incorporated by reference for the purpose of describing and disclosing, for example, the designs and methodologies that are described in the publications which might be used in connection with the presently described invention. The publications listed or discussed above, below and throughout the text are provided solely for their disclosure prior to the filing date of the present application. Nothing herein is to be construed as an admission that the inventors are not entitled to antedate such disclosure by virtue of prior invention.

### A. Detection of neuraminidase genes nanA, nanB and nanC of S. pneumoniae

NanA and NanB have been considered to be virulence factors of *S. pneumoniae;* however, NanC remains poorly understood. The *nanC* gene (Seq. ID No. 3) was found in the genome of a serotype 14 strain that was isolated from a child with HUS. In this invention, it is confirmed that the *S. pneumoniae* neuraminidase genes *nanC* (Seq. ID No. 3) as well as *nanA* (Seq. ID No. 1) and *nanB* (Seq. ID No. 2) are important virulence factors. Three primer sets for polymerase chain reaction (PCR) are designed for the detection and cloning of the neuraminidase genes that are *nanA* (Seq. ID No. 1), *nanB* (Seq. ID No. 2), and *nanC* (Seq. ID No. 3), based on the genomic sequence of *S. pneumoniae* strain CGSP14 with NCBI accession number NC_010582.

### A1. Primers designed to amplify nanA, nanB and nanC

Three primer sets used for PCR-amplification of *nanA* (Seq. ID No. 1), *nanB* (Seq. ID No. 2) and *nanC* (Seq. ID No. 3) are designed with two purposes: one is provided for gene detection, and the other for gene cloning into an expression vector as described later.
1. For *nanA* amplification based on Seq. ID No. 1:
   NanA-ATG-Kpn1:5'-AGATCTGGGTACC**ATGTCTTATTTCAGAA ATCG**
   NanA-TAA-Xho1: 5'-TGGTG CTCGA G**TTGTTCTCTCTTTTTCCCT A**
   The expected size of amplicon is 2964 bp long.
2. For *nanB* amplification based on Seq. ID No. 2:
   NanB-ATG-Kpn1:5'-AGATCTGGGTACC**ATGA AAAGAGGTCTTTA**
   NanB-TAA-Xho1: 5'-TGGTG CTCGA G**TTTTGTTAA ATCATTAATT TC**
   The expected size of amplicon is 2115 bp long.
3. For *nanC* amplification based on Seq. ID No. 3:
   NanC-ATG-Kpn1:5'-AGATCTGGGTACC**ATGAAAAAAAAT ATTAAACA**
   NanC-TAA-Xho1: 5'-TGGTG CTCGA G**ATTCTTTTTCAGATCTTCAA**
   The expected size of amplicon is 2244 bp long.
4. For *nanA4* amplification based on Seq. ID No. 1, in which its deduced amino acid sequence of resulting amplicon is listed as Seq. ID No. 4:
   NanA4- Kpn1:5'-AGATCTGGGTACC **AGCCAGCCTTCTTCAGAGAC**
   NanA4- Xho1: 5'-TGGTG CTCGA G **TGATAGGGTATAGGCATTTT**
      The expected size of amplicon is 2100 bp long, and the number of its deduced amino acids (aa) is 700.
5. For *nanB3* amplification based on Seq. ID No. 2, in which its deduced amino acid sequence of resulting amplicon is listed as Seq. ID No. 5:
   NanB3- Kpn1:5'-AGATCTGGGTACC **ATGAATAAAAGAGGTCTTTA**
   NanB3- Xho1: 5'-TGGTG CTCGA G **TTGAGTAGAATCTCCTGATT**
   The expected size of amplicon is 720 bp long, and the number of its deduced amino acids (aa) is 240.
6. For *nanC4* amplification based on Seq. ID No. 3, in which its deduced amino acid sequence of resulting amplicon is listed as Seq. ID No. 6:
   NanC4- Kpn1:5'-AGATCTGGGTACC **ATGAAAAAAAATATTAAACA**
   NanC4- Xho1: 5'-TGGTG CTCGA G **ACTTGTAGCAATATTAATTT**
The expected size of amplicon is 975 bp long, and the number of its deduced amino acids (aa) is 325.

In these primer sets, the bold sequences based on the neuraminidase genes are designed for the clonings of full-length and partial genes; the underlined sequences GGTACC and CTCGAG are the *Kpn*I and *Xho*I recognition sites, respectively, which are built in for cloning into an expression vector; and the plain sequences are extra-sequences which are generated for efficient digests by *Kpn*I and *Xho*I*.*

### B. Biofunctional assays of neuraminidases NanA, NanB and NanC of S. pneumoniae strain CGSP14

In order to analyze the biofunction of neuraminidases in this invention, the recombinant NanA, NanB, and NanC of *S. pneumoniae* strains CGSP14 were cloned by using the primer sets as described in the previous section, and also characterized for their features. These biofunctional assays characterized include the exposure of the Thomsen-Friedenrich antigen (TA) and substrate specificity as the follows.

### B1. Cloning, expression, and purification of recombinant NanA, NanB, and NanC

Referred to Figures 1A-1C, full-length- (Figure 1A) and partial- sequences (Figures 1B and 1C) of genes *nanA* (Sequence ID No. 1), *nanB* (Seq. ID No. 2), and *nanC* (Seq. ID No. 3) based on the genomic sequence of *S. pneumoniae* strain CGSP14 with NCBI accession number NC_010582 were PCR-amplified and cloned into an expression vector, such as pET29b (NOVAGEN, MERCK, Darmstadt, Germany), using *Kpn*I and *Xho*I as cloning sites. To narrow down the sufficient antigenic regions in neuraminidases that can be used to immunize animals for specific anti-sera that can effectively inhibit neuraminidase activities, subclonings of neuraminidase genes were performed. The resulting sub-clones containing the regions of LamG superfamily (lectin domain) and sialidase superfmaily, including 501∼780 amino acids (aa) of NanA, 501∼620 aa of NanA, 1∼325 aa of NanA, 80∼780 aa of NanA, 382∼473 aa of NanC, 578∼663 aa of NanC, 1∼240 aa of NanB, and 1∼325 aa of NanC, are denoted as NanA1, NanA2, NanA3, NanA4, NanC1, NanC2, NanB3, and NanC4, respectively. The amino acid sequences of three resulting sub-clones with better efficacy as described in the section of "Inhibition of neuraminidase activity by antibodies or immunized antisera" are NanA4, NanB3, and NanC4, listed in Seq. IDs No. 4, No. 5, and No. 6, respectively. The recombinant proteins, thus, can be inducibly over-expressed by the supplement of isopropyl β-D-1-thiogalactopyranoside (IPTG, 1 g/mL) in any Gram-negative bacteria, such as *Escherichia coli* BL21 (DE3). *E. coli* clones were cultured in Luria-Bertani (LB) broth at 37°C for 4 hours with IPTG induction, where the original culture was 1/100 dilution with LB broth prior to IPTG induction. The recombinant NanA, NanB and NanC with histidine tag may be easily purified according to the manufacturer's instructions for any kinds of Ni²⁺ affinity chromatography, such as Nickel-Chelating Resin (Invitrogen, Carlsbad, CA, USA).

### B2. TA exposure activities on cells used to confirm the function of recombinant NanA, NanB and NanC

Referred to Figures 2A-2D, the TA exposure activities of the recombinant neuraminidases were tested.

Lectins are usually used to recognize glycoconjugate residues (such as TA antigen) on cells. Fluorescein-labeled peanut agglutinin (PNA; Vector Laboratories, Inc., Burlingame, CA 94010, U.S.A.) is commonly used to detect TA on cells. Fluorescein-labeled *Sambucus Nigra* lectin (SNA; Vector Laboratories, Inc., Burlingame, CA 94010, U.S.A.) and biotinylated *Maackia Amurensis* lectin II (MAL II; Vector Laboratories, Inc., Burlingame, CA 94010, U.S.A.) are applied to recognize α2-6 and α2-3 sialyl linkages, respectively.

For the detection of the glycoconjugates on red blood cell (RBC), freshly collected blood samples from healthy volunteers were used to prepare the RBC fraction according to the method described in AABB Technical Manual, 14th Edition. RBC (3x10⁷ cells/mL), A549 (human epithelial lung cell line; ATCC^{®} Number: CCL-185™) and HK-2 (human kidney 2 cell line; ATCC^{®} Number: CRU-2190™) cells (1x10⁶ cells/mL) were cultured in Dulbecco's modified Eagle's medium (DMEM) plus Ham F12 medium, and treated with neuraminidase NanA, NanB or NanC (1 µg for RBC; 0.1 µg for A549 and HK-2). The mixture was incubated at 37°C for 1-2 hours For flow cytometric (FACScan, Becton Dickinson, USA) analysis, 10,000-20,000 cells were used, and cell labeling with each of lectins, including PNA, SNA and MAL II was done at 0∼10°C, preferably 4°C, for one hour, rather than higher temperature (such as 37°C) and longer time period (such as overnight) to cause cell agglutination, which would jam flow analysis. Biotinylated MAL II labeling can be observed by using fluorescein-conjugated streptavidin. Furthermore, to observe for cell agglutination by microscopy, 20 µl aliquots of lectin-labeled RBC were incubated at 37°C for 30 minutes.

As shown in a previous report, TA exposure on RBC, platelets and glomeruli is mediated by the secreted NanA in pneumococcal infection. To proof whether NanC was also a potential virulence factor associated with HUS, the ability of NanC was analyzed to expose TA on cells. When RBC, A549 and HK-2 cells were treated with the recombinant NanB and NanC, TA exposure was detected (Figures 2A, 2B, and 2C). On RBC, the TA exposure activity of NanA had shown to reach a plateau when NanA used was more than 0.01 µg. Thus, 0.01-µg NanA was used to compare with 1-µg NanB and 1-µg NanC. The results showed that the activity of NanA was 9.4 x 10² and 5.3 x 10² times higher than those of NanB and NanC, respectively. When lectin-PNA was used to verify TA exposure on RBC, NanA-treated RBC showed larger aggregates under microscopic examination, compared to the treatments by NanB and NanC (Figure 2D), whereas no agglutination was present with PNA in the case of untreated RBC. NanA activity shown on A549 cells was 2.2 and 3.3 times higher than NanB and NanC, respectively, while NanA activity on HK-2 cells was 1.5 times higher than both NanB and NanC.

### C. Protection by immunization using recombinant NanA, NanB, and NanC as antigens

In order to develop an ideal vaccine against pneumococcal infection, particularly to be a universal protein vaccine, three pneumococcal neuraminidases are chosen as a vaccine material to immunize mice, while PPV23 vaccine was used as a positive control. Meanwhile, the neuraminidase-immunized antisera were also applied for the inhibition assay against the neuraminidase activity.

### C1. Vaccination against S. pneumoniae in mice

Referred to Figures 3, 4 and 5, the recombinant NanA, NanB and NanC were used as the antigens to protect the mice against *S. pneumoniae* in mice in this invention. For comparison of vaccination, the individual or combination of neuraminidases NanA, NanB and NanC (10 µg/each enzyme, about 0.5-mg neuraminidase/kg body weight) were applied to immune mice (BALB/C, one month old) four times at 2-week interval prior to *S*. *pneumoniae* (3x10³ cfu) challenge, while 23-valent Pneumovax® (Merck Sharp & Dohme Corp., NJ08889, USA) polysaccharide vaccine (PPV23) and only phosphate buffered saline (PBS) were used as positive and negative controls, respectively. Freund's complete adjuvant for first time immunization and Freund's incomplete adjuvant for the last three immunizations were used with the ratio of 1:1 to the antigen(s).

To confirm the efficacy of mouse polyclonal anti-neuraminidase(s) antisera which were immunized by neuraminidase(s), enzyme-linked immunosorbent assay (ELISA) which is based on the antigen-antibody sandwich principle was performed. For ELISA test, the neuraminidase was first coated on an ELISA plate (Corning Incorporated, Corning, New York, USA). The anti-neuraminidase antiserum raised from mouse was then added to test how much antiserum was able to specifically bind on ELISA plate, and the antigen-antibody interaction was quantified by goat HRP-conjugated antimouse immunoglobulin G (IgG) as a secondary antibody (Millipore, Billerica, MA 01821, USA) and TMB/peroxide (R&D Systems, Minneapolis, MN, USA) as a color-developing substrate. The post-immune antisera against neuraminidase(s) from different groups of combinations were tested, while the control sera from the pre-immune and the negative control with only PBS plus Freund's complete/incomplete adjuvants were also examined (Figure 3). The value of antigen-antibody interaction was measured and presented logarithmically, as shown in Figure 3. The results showed that each value of neuraminidase-immunized antisera, compared to the control sera, was 3-4 logarithm folds increase, revealing that each of three neuraminidases is an ideal antigen for immunization (Figure 3).

For development of mouse vaccine against *S*. *pneumoniae,* the individual or combination of three neuraminidases, NanA, NanB and NanC with 10 µg of each enzyme, were applied to immunize nine mice (BALB/C, one month old) four times at 2-week interval, while PPV23 vaccine and a negative control (PBS + Freund's adjuvant) were taken for comparison. Thereafter, *S. pneumoniae* serotype 3 (3x10³ cfu) was injected in mice tails intravenously to challenge those immunized mice (Figure 4). Freund's complete adjuvant for the first time immunization and Freund's incomplete adjuvant for the last three immunizations were used to mix with the antigen(s) with the ratio of 1:1. Mouse survival rate (%) was determined during 14 days of feeding after four times neuraminidase immunization and a following challenge using *S. pneumoniae* serotype 3. As shown in Figure 4, vaccination tests showed that the group with the combination of three neuraminidases (NanA+NanB+NanC) presented the same 67% survival rate as that using the PPV23 vaccine, which value was the highest when compared to the other groups with one or two of three neuraminidases. In this invention, the combination containing three neuraminidases (NanA, NanB, and NanC) together was evaluted to be the best vaccine candidate for vaccination against *S. pneumoniae* infection in mice, and it also would be an appropriate candidate as a kind of universal protein vaccine.

To assess the protective effect of vaccination against different serotypes, BALB/c mice (n = 5) were immunized with combination of three neuraminidases NanA, NanB and NanC with 10 µg of each enzyme four times at 2-week interval, while PPV23 vaccine (n = 3) and a negative control (PBS + Freund's adjuvant, n = 3) were taken for comparison. Thereafter, mice were challenged with different serotypes *S*. *pneumoniae* serotype 3 (5x10⁶ cfu), serotype 6B (7x10⁸ cfu), serotype 14 (1x10⁸ cfu), serotype 15B (7x10⁸ cfu), serotype 19A (7x10⁸ cfu) and serotype 23F (1x10⁸ cfu). Serotypes 15B and 19A included in this study are the non-vaccine serotypes that have become common in PCV7 vaccinated populations. As shown in Figure 5, vaccination with combination of NanA, NanB and NanC caused significant increase in survival in a systemic infection model against different serotypes when compared to PPV23 vaccine and negative control, in serotype 3, 6B, 14, 15B, 19A and 23F survival was 60 %, 60 %, 20 %, 60 %, 40 % and 40 %, respectively. These results indicated that the vaccination with a combination of three neuraminidases can protect mice against different pneumococcal serotypes.

For the development of a vaccine against *S*. *pneumoniae,* the present invention uses one or more of the three neuraminidases as a vaccine composition, which can protect mice against different pneumococcal serotypes. The vaccine composition comprises recombinant pneumococcal neuraminidases, PBS, and Freund's adjuvant for immunization, wherein the recombinant pneumococcal neuraminidases include NanA, NanB, NanC, and sub-clones thereof. Freund's adjuvants are complete and/or incomplete Freund's adjuvants.

The procedure of making the vaccine composition includes the following steps: 1. The dosage of neuraminidases depending on the body weight is determined, and then one or more of NanA, NanB, and NanC (0.5 mg neuraminidase/per kg of body weight) is used. 2. PBS is used to adjust the concentration of recombinant pneumococcal neuraminidases, which has the same specific gravity with Freund's adjuvant 3. Then the same volume of pneumococcal neuraminidases solution in step 1 and Freund's adjuvant are used to form the vaccine composition.

The vaccine composition can be applied for mass production of pneumococcal neuraminidases enzyme, which can be commercialized. Pneumococcal neuraminidases can be enzymes that bind with specific substrates, for example: sialic acid, which can be used for substrate-specific combination and chemical reaction.

### C2. Inhibition of neuraminidase activity by antibodies or immunized antisera

As referred to Figures 6A to 8C, the inhibition assay was taken to test how efficient the neuraminidase activity can be inhibited by neuraminidase-specific antisera. The antisera against individual neuraminidase (NanA, NanB or NanC) were raised from rabbits, and then tested for their inhibitory effect on the activities of neuraminidases. NanC antiserum was purified by Protein A Sepharose beads (GE Healthcare) to increase its inhibition efficiency. Different amounts of neuraminidases in 10-µL PBS were pre-incubated with 10-µL immunized serum for 5 minutes. The antiserum-treated neuraminidase was mixed with RBC cells (4 × 10⁶ cells/mL) for 2 hours of incubation at 0∼10°C,preferably 4°C. Inhibition of neuraminidases (NanA, NanB, and NanC) activity by rabbit antisera was quantified by TA exposure on RBCs using flow cytometry, wherein the detection of TA exposure on RBC cells using FITC-labeled PNA lectin was described in previous section. If neuraminidase is neutralized by a specific anti-neuraminidase serum, the exposure of TA antigen on RBC cells and the value of fluorescence intensity will be reduced.

As shown on y-axis of Figures 6A-6C, the activities of NanB and NanC for TA antigen exposure were naturally weaker than that of NanA. NanA activity was completely inhibited by 10-µL anti-NanA antiserum when both 0.1-µg and 0.01-µg NanA were used, while only 20% activity was inhibted when 1-µg NanA was used (Figure 6A). The 1-µg NanB activity was completely inhibited by 10-µL anti-NanB antiserum. However, only 40% NanC activity was inhibited by 10-µL anti-NanC antiserum, but 90% NanC activity was inhibited by 30-µg purified anti-NanC antiserum (Figure 6C).

For cross-reactivity, anti-neuraminidase (including NanA, NanB and NanC) antisera were tested. Anti-NanA antiserum did not show inhibitory effect on the activity of NanB and NanC and vice versa. However, anti-NanB antiserum could inhibit NanC activity by 74%. Non-purified and purified anti-NanC antisera inhibited NanB activity by 40% and 76%, respectively.

The antisera from rabbit used to inhibit 50% neuraminidase activity were also assessed by titration test (data not shown). The dilution of anti-NanA antiserum for 50% NanA inhibition was 1, 8, and 32 for 1 µg, 0.1 µg and 0.01 µg, respectively. 50 % NanB (1 µg) activity with anti-NanB antiserum was inhibited by 16 folds of dilution. The dilutions of non-purified and purified anti-NanC antisera to inhibit 50% NanC (1 µg) activity were 1 and 8, respectively.

Similar to the test for the rabbit anti-neuraminidase antisera (as shown in Figures 6A-6C), the anti-neuraminidase antisera raised from mice were also shown to specifically inhibit neuraminidase activities (Figures 7A-7C). The 10-µL mouse Anti-NanA and anti-NanB antisera both enable to completely inhibit the activities of 0.1-µg NanA and 1-µg NanB, respectively. However, anti-NanC antiserum (10-µL) was only able to neutralize 50% activity of 1-µg NanC. The results indicated that the anti-sera raised from mice similar to those from rabbits can exhibit the inhibition of neuraminidase-mediated TA-exposure on RBCs.

To identify the neuraminidase domains that contain sufficient antigenic regions (protective epitopes) to immunize animals, a series of sub-clones of neuraminidases were constructed (as described in Figures 1B and 1C). Individual neuraminidases that included 0.0005-µg NanA, 0.005-µg NanB and 0.05-µg NanC, were used for the tests of inhibitions of neuraminidase activities by the raised antisera. Similar to the tests of the neuraminidase inhibitions using anti-neuraminidase antisera raised from both rabbits (as shown in Figures 6A-6C) and mice (as shown in Figures 7A-7C), the sub-clones possessing the sufficient antigenic regions that can immunize animal to generate antisera with effective inhibitions of neuraminidase activities equivalent to those raised by full length of neuraminidases are NanA4, NanB3, and NanC4, as illustrated in Figures 8A-8C. Moreover, NanC-specific antisera raised in mice immunized with the subclone NanC4 also showed cross-inhibition against NanB activity not just against NanC activity. The results revealed that three sufficient antigenic regions in neuraminidases were determined, including the NanA4 containing the lectin-binding (LamG) and sialidase superfamilies of NanA, the NanB3 containing lectin-binding domain superfamily of NanB, and the NanC4 containing lectin-binding superfamily of NanC, in which NanC4-specific antiserum can also exhibit cross-inhibition of NanB activity.

### C3. The influence of nanB and nanC mutations on the exposure of T-antigen on the mouse RBC in vivo

The *nanB* (Seq. ID No. 2) and *nanC* (Seq. ID No. 3) mutations were individually constructed by double crossing-over recombination mutagenesis. The chromosomal DNA of serotype 14 *S*. *pneumoniae* strain CGSP14 with a reference sequence of GenBank accession number of NC_010582 was used to amplify each of *nanB* (Seq. ID No. 2) and *nanC* (Seq. ID No. 3). The PCR amplified fragments were cloned into TA cloning vector (*RBC* Biosicience, Taiwan). The spectinomycin resistance gene *"spc"* (GI:1185605; accession number: CVU46202) was then inserted into the plasmids at *Msc*I and *Dra*I, respectively. The resulting plasmid that contained each of *nanB*::*spc* and *nanC*::*spc* was constructed, was transformed into strain 6B with the selection of spectinomycin. Transformation of *S*. *pneumoniae* was carried out as follows. Overnight *S*. *pneumoniae* cultures were diluted (1:50) using pH 6.8 Todd-Hewitt broth and 0.5% yeast extract (THY) supplemented with 1 mM CaCl₂ and 0.2% bovine serum albumin. The cultures were used until the early log phase, where a supplement with 10 mM NaOH, 1 mM CaCl₂, 0.2% BSA, 100 ng/ml CSP-1 and 10% horse serum were added, and followed by 15-min incubation at 37 °C. 2-3-µg plasmid DNA containing each of *nanB::spc* and *nanC::spc* was used to treat the cultures for 2 h at 37 °C under the supplement of 5% CO₂. The blood agar plates containing 350 µg/ml spectinomycin were used to select spectinomycin-resistant mutants. Each of the *nanB* (Seq. ID No. 2) and *nanC* (Seq. ID No. 3) mutations with gene replacement in *S. pneumoniae* was confirmed by PCR and DNA sequencing.

As referred to Figures 9A to 9F, the influence of *nanB* (Seq. ID No. 2) and *nanC* (Seq. ID No. 3) mutants on the exposure of T-antigen on RBC was examined. Each of wild type *S. pneumoniae, nanB* (Seq. ID No. 2) mutant and *nanC* (Seq. ID No. 3) mutant was used to intravenously challenge BALB/c mice, in which the total mouse number tested is 16. At specific time points, 1^{st} day (9A), 2^{nd} day (9B), 3^{rd} day (9C), 5^{th} day (9D) and 7^{th} day (9E), the RBC from tested mice were then collected, and examined for the T-antigen exposure by flow cytometer. Significantly lower FITC values were observed from the treatment with each of *nanB* and *nanC* mutants, compared to that with wild type strain 6B, indicating that the mutants caused less T-antigen exposure on RBC than wild type (p < 0.05), with the respective *p* values of 0.0485 and 0.0101 at the 5^{th} day after challenge, and with the respective *p* values of 0.0050 and 0.0025 at the 7^{th}-day after challenge. (9F) On the other hand, based on the results at the 7^{th} day after challenge of *S. pneumoniae,* the number of mice with positive T-antigen exposure on RBC challenged by each of *nanB* (Seq. ID No. 2) and *nanC* (Seq. ID No. 3) mutants is significantly less, compared to that of the mice challenged by wild type *S. pneumoniae,* with the *p* values of 0.0131 and 0.0325, respectively. The results indicated that NanB and NanC significantly causing T-antigen exposure on RBC are important virulence factors of *S. pneumoniae.* Thus, not just NanA, but also NanB and NanC are appropriate candidates as vaccine antigens.

Taken together, the results indicated that each of anti-neuraminidase antisera raised from both rabbit and mice enables to specifically inhibit or neutralize its corresponding neuraminidase activity; however, only anti-NanB and anti-NanC antisera have cross-protection abilities against each other. Although the inhibition of the anti-NanC antiserum was not as efficient as those anti-NanA and anti-NanB antisera, the combination of NanC with NanA and NanB is the best candidate as a vaccine.

Having described the invention by the description and illustrations above, it should be understood that these are exemplary of the invention and are not to be considered as limiting. Accordingly, the invention is not to be considered as limited by the foregoing description, but includes any equivalents.

## Claims

1. A protein vaccine composition to prevent pneumococcal diseases in a subject comprising:
recombinant pneumococcal neuraminidases, phosphate buffered saline (PBS), and Freund's adjuvant for immunization,
wherein the recombinant pneumococcal neuraminidases include NanA, NanB, NanC, and sub-clones thereof, wherein PBS is used to adjust the concentration of the recombinant pneumococcal neuraminidases, which has the same specific gravity with adjuvant, then the same volume of the recombinant pneumococcal neuraminidases solution and adjuvant are used to form the vaccine composition.

2. A protein vaccine composition to prevent pneumococcal diseases in a subject of claim 1, wherein adjuvants are complete or incomplete Freund's adjuvants.

3. A protein vaccine composition to prevent pneumococcal diseases in a subject of claim 1, wherein said pneumoniae comprises all kinds of *Streptococcus pneumoniae (S. pneumoniae)* serotypes.

4. A protein vaccine composition to prevent pneumococcal diseases in a subject of claim 1, wherein the pneumococcal genes include *nanA* (Seq. ID No. 1), *nanB* (Seq. ID No. 2), *nanC* (Seq. ID No. 3), and sub-clones theof, wherein the sub-clones include *nanA4* (Seq. ID No. 4, belonging to a *nanA* sub-clone), *nanB3* (Seq. ID No. 5, belonging to a *nanB* sub-clone), and *nanC4* (Seq. ID No. 6, belonging to a *nanC* sub-clone).

5. A protein vaccine composition to prevent pneumococcal diseases in a subject of claim 1, wherein the sub-clones of pneumococcal neuraminidases are the narrowed-down recombinants with full length of neuraminidases-equivalent efficacy for immuno-inhibition of neuraminidase activity, in which the sub-clones of neuraminidases comprise a region of LamG and sialidase superfamilies of NanA and a region of LamG superfamily of each NanB and NanC.

6. A protein vaccine composition to prevent pneumococcal diseases in a subject of claim 1, wherein the vaccine composition can be used to prevent pneumococcal infection.

7. A protein vaccine composition to prevent pneumococcal diseases in a subject of claim 1, wherein the pneumococcal neuraminidases can be produced as pneumococcal neuraminidases enzymes, wherein the pneumococcal neuraminidases can be enzymes that bind with specific substrates , which can be used for substrate-specific combination and chemical reaction.

8. A protein vaccine composition to prevent pneumococcal diseases in a subject of claim 7, wherein the specific substrate is sialic acid.

9. A method for detecting the presence of any of the three neuraminidases thereof in the *S. pneumoniae*-infected samples using anti-neuraminidase antibodies, comprising steps of:
using antibodies against neuraminidases NanA, NanB, and NanC, wherein said antibodies are prepared from crude antisera or purified antibodies;
detecting the presence of *S*. *pneumoniae* strains in a sample including human subjects or biological samples, including pus, blood, and urine, wherein techniques used in detecting the presence of *S*. *pneumoniae* and its components using any kinds of techniques, comprising Western blotting, enzyme linked immunosorbent assay, immunofluorescence labeling, radioimmunoassay, immunoradiometric assay, amplifying pneumococcal genes in *S. pneumoniae*-infected samples by PCR ,and combination thereof,
wherein pneumococcal genes in *S. pneumoniae*-infected samples are amplified by PCR and said pneumococcal genes including *nanA* (Seq. ID No. 1), *nanB* (Seq. ID No. 2), *nanC* (Seq. ID No. 3), and sub-clones thereof are detected, wherein sub-clones include *nanA4* (Seq. ID No. 4, belonging to a *nanA* sub-clone), *nanB3* (Seq. ID No. 5, belonging to a *nanB* sub-clone), and *nanC4* (Seq. ID No. 6, belonging to a *nanC* sub-clone ).

10. A method of detecting inhibition of neuraminidase activity comprises steps of:
using antibodies generated from neuraminidases-immunized animals, wherein said antibodies are prepared from crude antisera or purified antibodies;
mixing said antibodies with more than one neuraminidases in a sample , wherein said sample is red blood cell (RBC) or any kind of cell lines; and
detecting the neuraminidase activity using any kind of devices or equipments,
wherein said activity is quantified by Thomsen-Friedenrich antigen (TA) exposure; and a fluorescein-labeled lectin is used to recognize TA antigen-exposed cell.

11. The method of detecting inhibition of neuraminidase activity of claim 10, further comprises a step of performing interaction of lectin and TA-exposed cell sample at a low temperature environment, wherein the temperature is 0∼10°C to prevent cell agglutination from jamming subsequent cytometric flow analysis.

12. The method of detecting inhibition of neuraminidase activity of claim 11, wherein the temperature is 4°C to prevent cell agglutination from jamming subsequent cytometric flow analysis.


SEQUENCE LISTING

<110> Chang Gung Medical Foundation, Linkou Branch
<120> DEVELOPMENT OF NEURAMINIDASE-BASED METHODS FOR DIAGNOSING AND
PREVENTING PNEUMOCOCCAL DISEASES
<160> 6

<210> 1
<211> 2943
<212> nanC DNA
<213> Streptococcus pneumoniae with NCBI accession number NC_010582

<400> 1
atgtcttatt tcagaaatcg ggatatagat atagagagga tcagtatgaa tcggagtgtt 60
caagaacgta agtgtcgtta tagcattagg aaactatcgg taggagcggt ttctatgatt 120
gtaggagcag tggtatttgg aacgtctcct gttttagctc aagaaggggc aagtgagcaa 180
cctctggcaa atgaaactca actttcgggg gagagctcaa ccctaactga tacagaaaag 240
agccagcctt cttcagagac tgaactttct ggcaataagc aagaacaaga aaggaaagat 300
aagcaagaag aaaaaattcc aagagattac tatgcacgag atttggaaaa tgtcgaaaca 360
gtgatagaaa aagaagatgt tgaaaccaat gcttcaaatg gtcagagagt tgatttatca 420
agtgaactag ataaactaaa gaaacttgaa aacgcaacag ttcacatgga gtttaagcca 480
gatgccaagg ccccagcatt ctataatctc ttttctgtgt caagtgctac taaaaaagat 540
gagtacttca ctatggcagt ttacaataat actgctactc tagaggggcg tggttcggat 600
gggcaacagt tttacggtaa ttacaacgat gcacccttaa aagttaaacc aggtcagtgg 660
aattctgtga ctttcacagt tgaaaaaccg acagcagaac tacctaaagg ccgagtgcgc 720
ctctacgtaa acggggtatt atctcgaaca agtctgaaat ctggcaattt cattaaagat 780
atgccagatg taacgcatgt gcaaatcgga gcaaccaagc gtgccaacaa tacggtttgg 840
gggtcaaatc tacagattca gaatctcact gtgtataatc gtgctttaac accagaagag 900
gtacaaaaac gtagtcaact ttttaaacgc tcagatttag aaaaaaaact acctgaagga 960
gcggttttaa cagagaaaac ggacatattc gaaagcgggc gtaacggtaa accaaataaa 1020
gatggaatca agagttatcg tattccagca cttctcaaga cagataaagg aactttgatc 1080
gcaggtgcag atgaacgccg tctccattcg agtgactggg gtgatatcgg tatggtcatc 1140
agacgtagtg aagataatgg aaagacatgg ggagataagg tggttatctc caatcttcga 1200
gataatcctg aagctaaaga tcctgctgcg ccatcgcctc taaatattga tatggttttg 1260
gttcaagacc cgacaacaaa gagaatcttc tcaatttatg atatgttccc agaaggtcga 1320
gcagtttttg gaatgccaaa aacacctgaa aaagcttatg aaaagatagg ggataaaact 1380
tatcaaatct tgtataaaca aggagagtct ggtcattata ctgttcgtga gaatggagaa 1440
gtgtataatg cacaaaatca aaagacggat tatcgtgttg tagtgaatcc aacagaacct 1500
ggctatagag ataaaggaaa tctttacaaa ggtcaggaat tgattggaaa tatctatttt 1560
gcacacagta caaaaaatcc atttagagta gccaatacga gctatctatg gatgtcatat 1620
agtgacgatg atggtaaaac ttggtctgca ccgagagaca ttactccagg tcttcgcaag 1680
gattggatga agttcctagg aacaggtcct ggaacaggaa ttgtacttcg gaatggccct 1740
cacaagggac ggattttgat accggtttat acgactaata atgtatctca cttaaatggc 1800
tcgcaatctt ctcgtgtcat ctattcagat gatcatggaa aaacttggca tgctggagaa 1860
gcggtcaacg ataaccgtca ggtagacggt caaaagatcc actcttctac gatgaacaat 1920
gaacgtgcgc aaaatacaga atcaacggtg gtacaactaa acaatggaga tgttaaactc 1980
tttatgcgtg gtttgactgg agatcttcag gttgctacaa gtaaagacgg aggagtgact 2040
tgggagaagg atatcaaacg ttatccacag gttaaagatg tctatgttca aatgtctgct 2100
atccatacga tgcacgaagg aaaagaatac atcatcctca gtaatgcagg tggaccgaaa 2160
cgtgaaaatg ggatggtcca cttggcacgt gtcgaagaaa atggtgagtt gacttggctc 2220
aaacacaatc caattcaaaa aggagagttt gcctataatt cgctccaaga attaggaaat 2280
ggggagtatg gtatcttgta tgaacatact gaaaaaggac aaaatgccta taccctatca 2340
tttagaaaat ttaattggga ctttttgagc aaagatctga tttctcctac cgaagcgaaa 2400
gtgaagcgaa ctagagagat gggcaaagga gagatgggca aaggagttat tggcttggag 2460
ttcgactcag aagtattggt caacaaggct ccaacccttc aattggcaaa tggtaaaaca 2520
gcgactttcc taacccagta tgatagcaag accttgttgt ttgcagtaga taaggaagat 2580
atcggacagg aaattattgg tatagctaaa ggaagcatcg aaagtatgca taatcttcct 2640
gtaaatctag caggtgccag agttcctggc ggagtaaatg gtagcaaagc agcggtgcat 2700
gaagttccag aatttacagg gggagttaat ggtacagagc cagctgttca tgaaatcgca 2760
gagtataagg gatctgattc gcttgtaact cttactacaa aagaagatta tacttacaaa 2820
gctcctcttg ctcagcaggc acttcctgaa acaggaaaca aggagagtga cctcctagct 2880
tcactaggac taacagcttt cttccttggt ctgtttacgc tagggaaaaa gagagaacaa 2940
taa 2943

<210> SEQ ID NO 2
<211> 2094
<212> nanB DNA
<213> Streptococcus pneumoniae with NCBI accession number NC_010582

<400> 2
atgaataaaa gaggtcttta ttcaaaacta ggaatttctg ttgtaggcat tagtctttta 60
atgggagttc ccactttgat tcatgcgaat gaattaaact atggtcaact gtccatatct 120
cctatttttc aaggaggttc atatcaactg aacaataaga gtatagatat cagctctttg 180
ttattagata aattgtctgg agagagtcag acagtagtaa tgaaatttaa agcagataaa 240
ccaaactctc ttcaagcttt gtttggccta tctaatagta aagcaggctt taaaaataat 300
tacttttcaa ttttcatgag agattctggt gagataggtg tagaaataag agacgcccaa 360
aagggaataa attatttatt ttctagacca gcttcattat ggggaaagca taaaggacag 420
gcagttgaaa atacactagt atttgtatct gattctaaag ataaaacata cacaatgtat 480
gttaatggaa tagaagtgtt ctctgaaaca gttgatacat ttttgccaat ttcaaatata 540
aatggtatag ataaggcaac actaggagct gttaatcgtg aaggcaagga acattacctc 600
gcaaaaggaa gtattgatga aatcagtcta tttaacaaag caattagtga tcaggaagtt 660
tcaaatattc ccttgtcaaa tccatttcag ttaattttcc aatcaggaga ttctactcaa 720
gctaactatt ttagaatacc gacactatat acattaagta gtggaagagt tctatcaagt 780
attgatgcac gttatggtgg gactcatgat tctaaaagta agattaatat tgccacttct 840
tatagtgatg ataatgggaa aacgtggagt gagccaattt ttgctatgaa gtttaatgac 900
tatgaggagc agttagttta ctggccacga gataataaat taaagaatag tcaaattagt 960
ggaagtgctt cattcataga ttcatccatt gttgaagata aaaaatctgg gaaaacgata 1020
ttactagctg atgttatgcc tgcgggtatt ggaaataata atgcaaataa agccgactca 1080
ggttttaaag aaataaatgg tcattattat ttaaaactaa agaagaatgg agataacgat 1140
ttccgttata cagttagaga aaatggtgtc gtttatgatg aaacaactaa taaacctaca 1200
aattatacta taaatgataa gtatgaagtt ttggagggag gaaagtcttt aacagtcgaa 1260
caatattcgg ttgattttga tagtggctct ttaagagaaa aacataatgg aaaacaggtc 1320
cctatgaatg ttttctacaa agattcgtta tttaaagtga ctcctactaa ttatatagca 1380
atgacaacta gtcagaatag aggagagagt tgggaacaat ttaagttgtt gcctccgttc 1440
ttaggagaaa aacataatgg aacttactta tgtcccggac aaggtttagc attaaaatca 1500
agtaacagat tgatttttgc aacatatact agtggagaac taacctatct catttctgat 1560
gatagtggtc aaacatggaa gaaatcctca gcttcaattc cgtttaaaaa tgcaacagca 1620
gaagcacaaa tggttgaact gagagatggt gtgattagaa cattctttag aaccactaca 1680
ggtaagatag cttatatgac tagtagagat tctggagaaa catggtcgga agtttcgtat 1740
attgatggaa tccaacaaac ttcatatggc acacaagtat ctgcaattaa atactctcaa 1800
ttaattgatg gaaaagaagc agtcattttg agtacaccaa attctagaag tggccgtaag 1860
ggaggtcaat tagttgtcgg tttagtcaat aaagaagatg atagtattga ttggaaatac 1920
cactatgata ttgatttgcc ttcgtatggt tatgcctatt ctgcgattac agaattgcca 1980
aatcatcaca taggtgtact gtttgaaaaa tatgattcgt ggtcgagaaa tgaattgcat 2040
ttaagcaatg tagttcagta tatagatttg gaaattaatg atttaacaaa ataa 2094

<210> SEQ ID NO 3
<211> 2223
<212> nanC DNA
<213> Streptococcus pneumoniae with NCBI accession number NC_010582

<400> 3
atgaaaaaaa atattaaaca atacgtaacc ttaggtactg cagtggtatt atcagcattt 60
gttgctaact cagttgcagc tcaggagact gaaacttctg aagtatcaac accagagttg 120
gtgcaacctg ttgcaccaac gactccgatt tcggaagtac aacctaaatc gggtaactct 180
tcggaagtta ctgtacaacc tagaacagtc gaaactactg ttaaggatcc atcttctgca 240
acggaagaaa ctcctgtctt agaaaaaaat aatgttactt taacaggggg cggagaaaat 300
gttactaatg agttaaagga taaatttact agcggtgact ttactgtagt gattaagtac 360
aatcagtcta gtgagaaagg cttacaagct ctgtttggaa tatctaattc taaacccggt 420
caacaaaata gttatgtaga tgtgttcctt agagacaatg gtgaattggg aatggaagcg 480
cgtgatactt cttccaataa aaatcaccta gtatccagac ctgcttcagt ttggggtaag 540
tacaaacaag aagctgtgac taacactgtt gcagtagtag cagattcagt caaaaaaaca 600

tattctttat atgcaaatgg tacaaaagta gtagaaaaga aagtggataa cttcttaaac 660
atcaaggata ttaaaggtat tgattactat atgcttgggg gagtgaaacg tgcaggaaaa 720
acggcgtttg gttttaacgg aacactagaa aatatcaaat tctttaatag tgcattggat 780
gaagaaactg ttaaaaagat gacaacaaac gctgttactg gacatttaat ttatacggct 840
aatgatacaa caggttctaa ctatttccgt attccagttc tgtatacttt tagcaatggt 900
cgggtatttt caagcattga cgctcgttac ggcggaactc atgatttttt gaataaaatt 960
aatattgcta caagttatag tgatgataat ggtaagacat ggactaaacc aaaattaaca 1020
ttggcattcg atgattttgc gccagtacca ttagaatggc cttgtgatgt tggtggacgt 1080
gacttacaaa tcagcggtgg tgcaacctat attgactctg ttattgttga aaaaaataac 1140
aaacaagtac tcatgtttgc ggatgtgatg cctgctggag taagttttag agaagctact 1200
agaaaagatt caggttataa acaaattgat ggtaattatt accttaaatt aaagaaacaa 1260
ggtgatactg attacaatta tactattcgt gagaatggta ctgtatacga cgatcgtacc 1320
aacagaccaa ctgaattttc agtagataaa aatttcggta ttaaacaaaa tggtaattat 1380
ttgacagtag agcaatattc ggtttcattt aaaaataata aaaagactga atatcgtaat 1440
gggactcacg tccatatgaa tatcttctac aaagatgcat tgttcaaagt agtgccaacg 1500
aactatattg catatatttc tagcaatgat catggcgaat cttggtctgc accaacttta 1560
ttacctccta taatgggact taatcggaat gcgccttatt tgggtcctgg acgtggaatc 1620
attgaaagct caactggacg tattcttatt ccgtcttaca ctggtaaaga gtctgcattc 1680
atttatagtg acgataatgg agcatcttgg aaagttaaag ttgtgccact tccttctagt 1740
tggtcagcag aagcacaatt tgtagaattg agtccaggag taattcaagc atacatgcgt 1800
acaaataatg gtaaaattgc atatttaaca agtacagacg caggtactac ttggagtgca 1860
ccggaatatt tgaaatttgt ttcaaatcca agttatggaa cacaattatc aatcatcaat 1920
tatagtcaac tgattgatgg taaaaaggct gtcattttaa gtactccaaa ctccacaaat 1980
ggtcgtaaac acggacaaat ttggattggt ctaattaatg atgataatac aattgattgg 2040
cgttatcatc acgacgttga ttatagtaac tatggatact catattcaac attgacagag 2100
ttaccaaatc atgaaattgg attgatgttt gaaaaatttg attcatggtc tcgtaatgaa 2160
cttcatatga aaaatgttgt accatatata acatttaaga ttgaagatct gaaaaagaat 2220
taa 2223

<210> SEQ ID NO 4
<211> 700
<212> NanA4
<213> a subclone of NanA protein
<214> Streptococcus pneumoniae nanA-encoded protein with NCBI Reference
Sequence accession number: YP_001836382

<220> The 700-amino acid sequences of the subclone nanA4-encoded protein
shown below are translated from the region of nucleotide sequences of
241~2340 in nanA.

<400> 4
Ser Gln Pro Ser Ser Glu Thr Glu Leu Ser Gly Asn Lys Gln Glu Gln
1               5                   10                  15
Glu Arg Lys Asp Lys Gln Glu Glu Lys Ile Pro Arg Asp Tyr Tyr Ala
            20                  25                  30
Arg Asp Leu Glu Asn Val Glu Thr Val Ile Glu Lys Glu Asp Val Glu
        35                  40                  45
Thr Asn Ala Ser Asn Gly Gln Arg Val Asp Leu Ser Ser Glu Leu Asp
    50                  55                  60
Lys Leu Lys Lys Leu Glu Asn Ala Thr Val His Met Glu Phe Lys Pro
65                  70                  75                  80
Asp Ala Lys Ala Pro Ala Phe Tyr Asn Leu Phe Ser Val Ser Ser Ala
                85                  90                  95
Thr Lys Lys Asp Glu Tyr Phe Thr Met Ala Val Tyr Asn Asn Thr Ala
            100                 105                 110
Thr Leu Glu Gly Arg Gly Ser Asp Gly Gln Gln Phe Tyr Gly Asn Tyr
        115                 120                 125
Asn Asp Ala Pro Leu Lys Val Lys Pro Gly Gln Trp Asn Ser Val Thr
    130                 135                 140
Phe Thr Val Glu Lys Pro Thr Ala Glu Leu Pro Lys Gly Arg Val Arg
145                 150                 155                 160

Leu Tyr Val Asn Gly Val Leu Ser Arg Thr Ser Leu Lys Ser Gly Asn
                165                 170                 175
Phe Ile Lys Asp Met Pro Asp Val Thr His Val Gln Ile Gly Ala Thr
            180                 185                 190
Lys Arg Ala Asn Asn Thr Val Trp Gly Ser Asn Leu Gln Ile Gln Asn
        195                 200                 205
Leu Thr Val Tyr Asn Arg Ala Leu Thr Pro Glu Glu Val Gln Lys Arg
    210                 215                 220
Ser Gln Leu Phe Lys Arg Ser Asp Leu Glu Lys Lys Leu Pro Glu Gly
225                 230                 235                 240
Ala Val Leu Thr Glu Lys Thr Asp Ile Phe Glu Ser Gly Arg Asn Gly
                245                 250                 255
Lys Pro Asn Lys Asp Gly Ile Lys Ser Tyr Arg Ile Pro Ala Leu Leu
            260                 265                 270
Lys Thr Asp Lys Gly Thr Leu Ile Ala Gly Ala Asp Glu Arg Arg Leu
        275                 280                 285
His Ser Ser Asp Trp Gly Asp Ile Gly Met Val Ile Arg Arg Ser Glu
    290                 295                 300
Asp Asn Gly Lys Thr Trp Gly Asp Lys Val Val Ile Ser Asn Leu Arg
305                 310                 315                 320

Asp Asn Pro Glu Ala Lys Asp Pro Ala Ala Pro Ser Pro Leu Asn Ile
                325                 330                 335
Asp Met Val Leu Val Gln Asp Pro Thr Thr Lys Arg Ile Phe Ser Ile
            340                 345                 350
Tyr Asp Met Phe Pro Glu Gly Arg Ala Val Phe Gly Met Pro Lys Thr
        355                 360                 365
Pro Glu Lys Ala Tyr Glu Lys Ile Gly Asp Lys Thr Tyr Gln Ile Leu
    370                 375                 380
Tyr Lys Gln Gly Glu Ser Gly His Tyr Thr Val Arg Glu Asn Gly Glu
385                 390                 395                 400
Val Tyr Asn Ala Gln Asn Gln Lys Thr Asp Tyr Arg Val Val Val Asn
                405                 410                 415
Pro Thr Glu Pro Gly Tyr Arg Asp Lys Gly Asn Leu Tyr Lys Gly Gln
            420                 425                 430
Glu Leu Ile Gly Asn Ile Tyr Phe Ala His Ser Thr Lys Asn Pro Phe
        435                 440                 445
Arg Val Ala Asn Thr Ser Tyr Leu Trp Met Ser Tyr Ser Asp Asp Asp
    450                 455                 460
Gly Lys Thr Trp Ser Ala Pro Arg Asp Ile Thr Pro Gly Leu Arg Lys
465                 470                 475                 480
Asp Trp Met Lys Phe Leu Gly Thr Gly Pro Gly Thr Gly Ile Val Leu
                485                 490                 495
Arg Asn Gly Pro His Lys Gly Arg Ile Leu Ile Pro Val Tyr Thr Thr
            500                 505                 510
Asn Asn Val Ser His Leu Asn Gly Ser Gln Ser Ser Arg Val Ile Tyr
        515                 520                 525
Ser Asp Asp His Gly Lys Thr Trp His Ala Gly Glu Ala Val Asn Asp
    530                 535                 540
Asn Arg Gln Val Asp Gly Gln Lys Ile His Ser Ser Thr Met Asn Asn
545                 550                 555                 560
Glu Arg Ala Gln Asn Thr Glu Ser Thr Val Val Gln Leu Asn Asn Gly
                565                 570                 575
Asp Val Lys Leu Phe Met Arg Gly Leu Thr Gly Asp Leu Gln Val Ala
            580                 585                 590
Thr Ser Lys Asp Gly Gly Val Thr Trp Glu Lys Asp Ile Lys Arg Tyr
        595                 600                 605
Pro Gln Val Lys Asp Val Tyr Val Gln Met Ser Ala Ile His Thr Met
    610                 615                 620
His Glu Gly Lys Glu Tyr Ile Ile Leu Ser Asn Ala Gly Gly Pro Lys
625                 630                 635                 640
Arg Glu Asn Gly Met Val His Leu Ala Arg Val Glu Glu Asn Gly Glu
                645                 650                 655

Leu Thr Trp Leu Lys His Asn Pro Ile Gln Lys Gly Glu Phe Ala Tyr
            660                 665                 670
Asn Ser Leu Gln Glu Leu Gly Asn Gly Glu Tyr Gly Ile Leu Tyr Glu
        675                 680                 685
His Thr Glu Lys Gly Gln Asn Ala Tyr Thr Leu Ser
    690                 695                 700

<210> SEQ ID NO 5
<211> 240
<212> NanB3
<213> a subclone of NanB protein
<214> Streptococcus pneumoniae nanB-encoded protein with NCBI Reference
Sequence accession number: YP_001836377

<220> The 240-amino acid sequences of the subclone nanB3-encoded protein
shown below are translated from the region of nucleotide sequences of 1~720
in nanB.

<400> 5
Met Asn Lys Arg Gly Leu Tyr Ser Lys Leu Gly Ile Ser Val Val Gly
  1               5                  10                  15
Ile Ser Leu Leu Met Gly Val Pro Thr Leu Ile His Ala Asn Glu Leu
             20                  25                  30
Asn Tyr Gly Gln Leu Ser Ile Ser Pro Ile Phe Gln Gly Gly Ser Tyr
         35                  40                  45
Gln Leu Asn Asn Lys Ser Ile Asp Ile Ser Ser Leu Leu Leu Asp Lys
     50                  55                  60
Leu Ser Gly Glu Ser Gln Thr Val Val Met Lys Phe Lys Ala Asp Lys
 65                  70                  75                  80
Pro Asn Ser Leu Gln Ala Leu Phe Gly Leu Ser Asn Ser Lys Ala Gly
                 85                  90                  95
Phe Lys Asn Asn Tyr Phe Ser Ile Phe Met Arg Asp Ser Gly Glu Ile
            100                 105                 110
Gly Val Glu Ile Arg Asp Ala Gln Lys Gly Ile Asn Tyr Leu Phe Ser
        115                 120                 125
Arg Pro Ala Ser Leu Trp Gly Lys His Lys Gly Gln Ala Val Glu Asn
    130                 135                 140
Thr Leu Val Phe Val Ser Asp Ser Lys Asp Lys Thr Tyr Thr Met Tyr
145                 150                 155                 160
Val Asn Gly Ile Glu Val Phe Ser Glu Thr Val Asp Thr Phe Leu Pro
                165                 170                 175
Ile Ser Asn Ile Asn Gly Ile Asp Lys Ala Thr Leu Gly Ala Val Asn
            180                 185                 190
Arg Glu Gly Lys Glu His Tyr Leu Ala Lys Gly Ser Ile Asp Glu Ile
        195                 200                 205
Ser Leu Phe Asn Lys Ala Ile Ser Asp Gln Glu Val Ser Asn Ile Pro
    210                 215                 220
Leu Ser Asn Pro Phe Gln Leu Ile Phe Gln Ser Gly Asp Ser Thr Gln
225                 230                 235                 240

<210> SEQ ID NO 6
<211> 325
<212> NanC4 Protein
<213> a subclone of nanC DNA
<214> Streptococcus pneumoniae nanC-encoded protein with NCBI Reference
Sequence accession number: YP_001835570

<220> The 325-amino acid sequences of the subclone nanC4-encoded protein
shown below are translated from the region of nucleotide sequences of 1~975
in nanC.

<400> 6
Met Lys Lys Asn Ile Lys Gln Tyr Val Thr Leu Gly Thr Ala Val Val
1               5                   10                  15
Leu Ser Ala Phe Val Ala Asn Ser Val Ala Ala Gln Glu Thr Glu Thr
            20                  25                  30
Ser Glu Val Ser Thr Pro Glu Leu Val Gln Pro Val Ala Pro Thr Thr
        35                  40                  45
Pro Ile Ser Glu Val Gln Pro Lys Ser Gly Asn Ser Ser Glu Val Thr
    50                  55                  60
Val Gln Pro Arg Thr Val Glu Thr Thr Val Lys Asp Pro Ser Ser Ala
65                  70                  75                  80
Thr Glu Glu Thr Pro Val Leu Glu Lys Asn Asn Val Thr Leu Thr Gly
                85                  90                  95
Gly Gly Glu Asn Val Thr Asn Glu Leu Lys Asp Lys Phe Thr Ser Gly
            100                 105                 110
Asp Phe Thr Val Val Ile Lys Tyr Asn Gln Ser Ser Glu Lys Gly Leu
        115                 120                 125
Gln Ala Leu Phe Gly Ile Ser Asn Ser Lys Pro Gly Gln Gln Asn Ser
    130                 135                 140
Tyr Val Asp Val Phe Leu Arg Asp Asn Gly Glu Leu Gly Met Glu Ala
145                 150                 155                 160
Arg Asp Thr Ser Ser Asn Lys Asn His Leu Val Ser Arg Pro Ala Ser
                165                 170                 175
Val Trp Gly Lys Tyr Lys Gln Glu Ala Val Thr Asn Thr Val Ala Val
            180                 185                 190
Val Ala Asp Ser Val Lys Lys Thr Tyr Ser Leu Tyr Ala Asn Gly Thr
        195                 200                 205
Lys Val Val Glu Lys Lys Val Asp Asn Phe Leu Asn Ile Lys Asp Ile
    210                 215                 220
Lys Gly Ile Asp Tyr Tyr Met Leu Gly Gly Val Lys Arg Ala Gly Lys
225                 230                 235                 240
Thr Ala Phe Gly Phe Asn Gly Thr Leu Glu Asn Ile Lys Phe Phe Asn
                245                 250                 255
Ser Ala Leu Asp Glu Glu Thr Val Lys Lys Met Thr Thr Asn Ala Val
            260                 265                 270
Thr Gly His Leu Ile Tyr Thr Ala Asn Asp Thr Thr Gly Ser Asn Tyr
        275                 280                 285
Phe Arg Ile Pro  Val Leu Tyr Thr Phe Ser Asn Gly Arg Val Phe Ser
    290                  295                 300
Ser Ile Asp Ala Arg Tyr Gly Gly Thr His Asp Phe Leu Asn Lys Ile
305                 310                 315                 320

Asn Ile Ala Thr Ser
                325